# EUROPEAN PATENT SPECIFICATION

(11) **EP 2 167 045 B1**
(45) Date of publication and mention of the grant of the patent: **09.08.2017**
(21) Application number: 08770312.0
(22) Date of filing: 06.06.2008
(51) Int. Cl.: A61K 31/77, A61K 45/06, A61K 9/00, A61K 47/10, A61L 15/26, A61L 15/44, A61P 7/00, A61P 9/10, A61P 17/02, A61P 35/00

(54) **TOPICAL POLOXAMER FORMULATIONS FOR ENHANCING MICROVASCULAR FLOW: COMPOSITIONS AND USES THEREOF**
TOPISCHE POLOXAMER-FORMULIERUNGEN ZUR VERBESSERUNG DES MIKROVASKULÄREN DURCHFLUSSES: ZUSAMMENSETZUNGEN UND IHRE VERWENDUNG
FORMULATIONS DE POLOXAMÈRE TOPIQUES POUR AMÉLIORER L'ÉCOULEMENT MICROVASCULAIRE : COMPOSITIONS ET UTILISATIONS DE CELLES-CI

(30) Priority: 08.06.2007 US 933902 P
(43) Date of publication of application: 31.03.2010
(73) Proprietor: University of Virginia Patent Foundation, Charlottesville, VA 22903 (US)
(72) Inventor: KATZ, Adam, J., Charlottesville, VA 22911 (US); RODEHEAVER, George, Charlottesville, VA 22903 (US)
(74) Representative: Fyfe, Fiona Allison Watson
(86) International application number: PCT/US2008/066094
(87) International publication number: WO 2008/154368

(56) References cited:
- WO-A1-01/85845
- WO-A1-03/045452
- WO-A1-2008/103673
- US-A- 3 393 206
- US-A- 5 298 260
- US-A- 5 622 649
- US-A- 5 635 540
- US-A- 5 939 485
- US-A1- 2004 142 020
- US-A1- 2004 202 640
- US-A1- 2005 079 147
- US-A1- 2005 123 602
- US-A1- 2007 088 245
- US-B1- 6 649 702
- NALBANDIAN R M ET AL: "Artificial skin. II. Pluronic F-127 Silver nitrate or silver lactate gel in the treatment of thermal burns", JOURNAL OF BIOMEDICAL MATERIALS RESEARCH, WILEY, NEW YORK, NY, US, vol. 6, no. 6, 1 November 1972 (1972-11-01), pages 583-590, XP002451837, ISSN: 0021-9304
- NALBANDIAN R M ET AL: "PLURONIC F-127 GEL PREPARATION AS AN ARTIFICIAL SKIN IN THE TREATMENT OF THIRD-DEGREE BURNS IN PIGS", JOURNAL OF BIOMEDICAL MATERIALS RESEARCH, WILEY, NEW YORK, NY, US, vol. 21, no. 9, 1 September 1987 (1987-09-01), pages 1135-1148, XP002915634, ISSN: 0021-9304, DOI: 10.1002/JBM.820210907

## Description

### FIELD OF INVENTION

This invention relates generally to the use of a novel formulation of a surface active polymer to enhance blood flow and/or reduce inflammation in injured or diseased skin and other soft tissue.

### BACKGROUND OF THE INVENTION

Poloxamers are water-soluble triblock copolymers composed of hydrophilic polyethylene oxide (PEO) and hydrophobic polypropylene oxide (PPO) blocks linked together. The amphiphilic nature of these block copolymers can be varied by controlling the length of the PEO and/or PPO block components (Ahmed et al., 2001). Several members of this poloxamer family of chemicals (such as poloxamer 188 and 407) are known to be biocompatible and non-toxic to mammalian cells and tissues, making them useful for biomedical applications. These compounds are surface acting agents (i.e. "surfactants") and known to incorporate into or onto mammalian cell membranes, and thereby reduce protein adsorption and cell adhesion.

After cutaneous burn injury, an area of tissue 1-2 mm thick surrounding the wound is the site of a pronounced inflammatory response where blood flow is reduced. This "zone of stasis" undergoes progressive necrosis within 24-48 hours, resulting in an expansion of the burn wound. The three zones of thermal injury have been described by the changes in microvasculature surrounding the point of injury: a peripheral zone with hyperemia, a zone of stasis, and a zone of coagulation.¹ Effects on microvasculature in the zone of stasis can influence the resulting ischemia and tissue loss.² Microvascular damage has been shown to be reversible within the zone of stasis.^{3,4} Poloxamer-188 (a copolymer of polyoxyethylene and polyoxypropylene) has demonstrated hemorheologic and antithrombotic effects on blood flow through interactions with erythrocytes and fibrin.⁵⁻⁷ Also, intravenous poloxamer-188 has been shown to improve capillary blood flow in cutaneous burn wounds.⁸

Given intravenously, poloxamer-188 has been shown to improve microvascular blood flow via hemorheologic and anti-adhesive changes and to prevent cell death due to electrical injury *in vivo* and heat shock *in vitro.* In addition, poloxamer-188 is currently used as a wound cleanser.

The skin serves as a protective barrier against the environment. The skin serves as a barrier to infection and prevents the loss of water and electrolytes from the body. Thus, the loss of the integrity of large portions of the skin as a result of illness or injury can lead to major disability or even death.

Every year in the United States there are 1.1 million burn patients who require medical attention and 6.5 million patients are reported to have chronic skin ulcers caused by pressure, venous stasis, or diabetes mellitus. Thus, acceleration of skin wound healing has been an active area of medical research and improved designs of skin repair materials have been sought for decades.

There is a long felt need in the art for compositions and methods useful for treating injuries and wounds topically. The present invention satisfies these needs.

The following documents may be useful in the understanding of the present application.

US5635540 relates to stabilized topical pharmaceutical preparations based on the solubilization of a eutectic mixture of local anesthetic agents, in base form, which when combined in the presence of a surfactant and water produce a single phase hydrated polymer to be used in obtaining topical anesthesia. US5635540 also relates to a stabilized antimicrobial topical pharmaceutical preparation.

WO03045452 relates to medical dressings treated with one or more poloxamers which show reduced tendency to adhere to a wound interface on removal, thereby reducing traumatisation and promoting healing.

WO0185845 relates to hydrogels containing poloxamers which are capable of absorbing more water than other hydrogels, while retaining gel integrity for longer, and are useful as occlusive dressings.

US2005079147 relates to poloxamer compositions and the use thereof for the purpose of treating skin wounds on a human body.

US5298260 relates to iso-osmotic, hyperosmotic, or hypo-osmotic, pH balanced, thermoreversible gels for use as medical devices or vehicles for drug delivery to the skin of a mammal.

NALBANDIAN R M ET AL, "Artificial skin. II. Pluronic F-127 Silver nitrate or silver lactate gel in the treatment of thermal burns", JOURNAL OF BIOMEDICAL MATERIALS RESEARCH, WILEY, NEW YORK, NY, US, (19721101), vol. 6, no. 6, ISSN 0021-9304, pages 583 - 590, describes Pluronic F-127 as an ideal base for bactericidal agents in the treatment of full thickness wounds. In addition, the Pluronic F-127 dressing with silver nitrate or silver lactate showed promise as an effective biological artificial skin against electrolyte imbalance, heat loss, and bacterial invasion.

NALBANDIAN R M ET AL, "PLURONIC F-127 GEL PREPARATION AS AN ARTIFICIAL SKIN IN THE TREATMENT OF THIRD-DEGREE BURNS IN PIGS", JOURNAL OF BIOMEDICAL MATERIALS RESEARCH, WILEY, NEW YORK, NY, US, (19870901), vol. 21, no. 9, doi:10.1002/JBM.820210907, ISSN 0021-9304, pages 1135 - 1148, describes the possible therapeutic benefits of the use of Pluronic® Polyol F-127 as a substitute skin in standardized third-degree thermal bums.

US2004142020 relates to an anhydrous, hydrophilic wound dressing containing a superabsorbent polymer which, when applied to a wound site, absorbs wound fluid.

US2004202640 relates to the topical treatment of keloids, hypertrophic scars and burn scars by the use of a selected protein kinase C inhibitior, an effective penetrating agent selected from the lecithin organogel, or poloxamer 407 lecithin organogel and a polyacrylate polymer, carbomer, to eliminate the stickiness of the poloxamer 407.

WO2008103673 relates to compositions containing a surface active agent and a sub-lethal amount of an antimicrobial agent and methods for using such compositions.

### SUMMARY OF THE INVENTION

The present invention relates to a topical pharmaceutical composition comprising an effective amount of at least one surface active copolymer and optionally at least one additional therapeutic agent, wherein said at least one surface active copolymer is poloxamer 188 at 5% concentration, for use in the treatment of an injury, disease, or disorder characterised by decreased blood flow; wherein the said injury, disease, or disorder is selected from the group consisting of thermal injury, skin injury, soft tissue injury, non-healing skin wound, bums, acute wound, chronic wound, scrape, cut, incision, laceration, decubitis, pressure ulcer, chronic venous ulcer, venous stasis ulcer, diabetic ulcer, arterial ulcer, radiation ulcer, traumatic wound, open complicated non-healing wound, body piercing, bite wound, insect bite, insect sting, stab wound, gunshot wound, stretch injury, crush wound, compression wound, fracture, sprain, strain, stroke, infarction, aneurism, herniation, ischemia, fistula, dislocation, radiation, surgery, cell, tissue or organ grafting, and cancer.

Preferred embodiments of the invention are set forth in the dependent claims.

Associated methods are also described herein to aid in the understanding of the invention, but these do not form part of the claimed invention.

Examples or embodiments described herein which do not fall under the definition of the claims do not form part of the present invention.

The present disclosure is based on the discovery described herein that topical administration of a composition comprising a surface active copolymer such as a poloxamer is useful for treating decreased blood flow at a site associated with an injury. Herein disclosed are compositions and methods for treating decreased blood flow at the site of specific injuries or at sites associated with certain diseases and conditions. The treatment improves blood flow compared to no treatment.

Disclosed herein are formulations of poloxamers or other surface active agents for topical delivery to injured and diseased tissues and their ability to inhibit the decreased blood flow associated with the injuries, diseases, and disorders described herein. The compounds described herein can inhibit decreased blood flow by influencing blood flow characteristics such as flow rate, stasis, and sludging.

The present disclosure includes compositions comprising at least one surface active copolymer and methods of treating a site of injury (*e.g*., burn injury, chronic wounds, skin grafts or other injury to skin or soft tissue) or exposed soft tissue using the compositions. In one example, the surface active copolymers include poloxamers, meroxapols, and poloxamines. The disclosure includes a therapeutic composition comprising at least one surface active copolymer at about 1%-65% w/w. The therapeutic compositions may be formulated, for example, as liquids or as stable gels.

The present disclosure encompasses treatment of various injuries, diseases, and disorders which are characterized by decreased blood flow. These include thermal injury, skin injury, soft tissue injury, non-healing skin wound, bums, acute wound, chronic wound, scrape, cut, incision, laceration, decubitis, pressure ulcer, chronic venous ulcer, venous stasis ulcer, diabetic ulcer, arterial ulcer, radiation ulcer, traumatic wound, open complicated non-healing wound, body piercing, bite wound, insect bite, insect sting, stab wound, gunshot wound, stretch injury, crush wound, compression wound, fracture, sprain, strain, stroke, infarction, aneurism, herniation, ischemia, fistula, dislocation, radiation, surgery, cell, tissue or organ grafting, and cancer.

The present disclosure provides compositions and methods for enhancing blood flow to burn injuries by administering an effective amount of the compositions of the invention to a site of injury. The types of bums encompassed by the present disclsoure include thermal, radiation, chemical, electrical, steam, and sunburn. In one example, the burn is a thermal injury. In one example, the thermal injury is a cutaneous injury or an injury of the mesentery of the intestine. In one example, the composition comprises at least two surface active copolymers. In one example, the blood flow is in microvessels.

The present disclosure provides compositions and methods for enhancing blood flow for chronic wounds by administering an effective amount of the compositions of the invention to a site of a chronic wound. In one example, the blood flow is microvascular blood flow. Chronic wounds include, for example, venous stasis ulcers, diabetic wounds, arterial ulcers, and pressure ulcers.

The present disclosure provides compositions and methods for enhancing microvascular blood flow to skin or tissue following a surgical procedure, for example, skin grafts, microvascular surgery and tissue flaps, by topically administering an effective amount of the compositions of the invention to the site.

The present disclosure provides compositions and methods for reducing inflammation at a site of injury by administering a therapeutic composition of the invention to a site of injury in an amount effective to reduce inflammation at the site of injury. The present disclosure provides compositions and methods for enhancing microvascular blood flow at a site of injury by administering a therapeutic composition of the invention to a site of injury in an amount effective to enhance microvascular blood flow at the site of injury.

The therapeutic compositions of the invention have use in treatment of exposed soft tissue or various injuries, for example, thermal injuries, venous stasis ulcers, diabetic wounds, skin grafts, tissue flaps, microvascular surgery, pressure ulcers.

These and embodiments and aspects of the invention, which will become apparent during the following detailed description, have been achieved by discovery described herein that compositions comprising surface active copolymers, for example, poloxamer-188, enhance microvascular blood flow to and reduce inflammation in injured skin and exposed soft tissue. The compositions disclosed herein and of the invention are useful for tissue salvage by their ability to maximize microvascular blood flow and/or reduce inflammation.

The route of administration can vary depending on the formulation of the pharmaceutical composition being administered as well as on the site of injury, disease, or disorder being treated. The present disclosure encompasses any useful means of topical administration of the pharmaceutical compositions of the disclosure to treat the injuries, diseases, and disorders encompassed by the methods described herein. In one example, the compounds are administered via direct, topical, cutaneous, mucosal, nasal, inhalation, oral, and ophthalmic. The means for the administration includes a dressing material, extruder, aerosol, spray delivery, iontophoresis, a patch, and a transdermal patch.

The present disclosure further provides for administration of a compound or additional therapeutic agent of the invention as a controlled-release formulation.

The dosage of the active compound(s) being administered will depend on the condition being treated, the particular compound, and other clinical factors such as age, sex, weight, and health of the subject being treated, the route of administration of the compound(s), and the type of composition being administered (gel, liquid, solution, suspension, aerosol, ointment, lotion, cream, paste, liniment, etc.). It is to be understood that the present invention has application for both human and veterinary use.

The present disclosure further provides for administration of the pharmaceutical compositions of the disclosure at different times before and after an injury or surgical procedure, as well as varying the optional additional therapeutic agents and the surface active copolymers.

The present disclosure provides a therapeutic composition comprising at least one poloxamer or other surface active copolymer agent at a concentration ranging from about 1% to about 65% w/w. Examples of poloxamers include poloxamer-101, -105, -105 benzoate, -108, -122, -123, -124, -181, -182, -182 dibenzoate, -183, -184, -185, -188, -212, -215, -217, -231, -234, -235, -237, -238, - 282, -284, -288, -331, -333, -334, -335, -338, -401, -402, -403, and -407. According to the present invention, the poloxamer is poloxamer-188. In another example, the poloxamer is poloxamer-407.

The present disclosure provides that the pharmaceutical composition comprises PluroGel™ (PluroGen, Annapolis, Maryland).

The present disclosure provides, at least one of the surface active copolymers is a meroxapol. Exemplary meroxapols include meroxapol 105, 108, 171, 172, 174, 178, 251, 252, 254, 258, 311, 312, and 314.

The present disclosure provides, at least one of the surface active copolymers is a poloxamine. Exemplary poloxamines include poloxamine 304, 504, 701, 702, 704, 707, 901, 904, 908, 1101, 1102, 1104, 1301, 1302, 1304, 1307, 1501, 1502, 1504, and 1508.

In one embodiment, the therapeutic composition is formulated as a liquid or stable gel. The copolymer size may range, for example, from an Mₙ of about 600 to about 20,000. In another aspect, the copolymer size may range, for example, from an Mₙ of about 1,000 to about 10,000.

The present disclosure encompasses a composition comprising a poloxamer at about 0.1% to about 85% w/w, or about 1% to about 65%, or about 1% to about 50%, or about 5% to about 40%, or about 10% to about 40%. Other surface active copolymers can be used at these concentrations as well.

The surface active copolymers may be prepared at different temperatures depending on the type of formulation being prepared, the route of administration, the site of administration. In one example, the surface active copolymer is prepared at a temperature ranging from about 0°F to about 70°F. In another example, the surface active copolymer is prepared at a temperature ranging from about 5°F to about 50°F. In yet another example, the surface active copolymer is prepared at a temperature ranging from about 10°F to about 40°F.

The composition may further comprise an effective amount of at least one additional therapeutic agents which may be useful for the type of injury, disease, or disorder being treated. Additional therapeutic agents include anesthetic, analgesic, antimicrobial, steroid, growth factor, cytokine, and anti-inflammatory agents. Useful anesthetic agents include benzocaine, lidocaine, bupivocaine, dibucaine, mepivocaine, etidocaine, tetracaine, butanilicaine, and trimecaine.

In another aspect, the agent is at least one analgesic. In yet another aspect, the agent is an additional therapeutic drug.

In a further aspect, the additional therapeutic agent is an antimicrobial agent. In one aspect, the antimicrobial agent is an antibacterial agent. In another aspect, the antimicrobial agent is an antifungal agent. In yet another aspect, the antimicrobial agent is an antiviral agent. Antimicrobial agents useful in the practice of the disclosure include silver sulfadiazine, Nystatin, Nystatin/triamcinolone, Bacitracin, nitrofurazone, nitrofurantoin, a polymyxin (*e.g*., Colistin, Surfactin, Polymyxin E, and Polymyxin B), doxycycline, antimicrobial peptides (*e.g*., natural and synthetic origin), Neosporin (i.e., Bacitracin, Polymyxin B, and Neomycin), Polysporin (i.e., Bacitracin and Polymyxin B). Additional antimicrobials include topical antimicrobials (i.e., antiseptics), examples of which include silver salts, iodine, benzalkonium chloride, alcohol, hydrogen peroxide, and chlorhexidine. It may be desirable for the antimicrobial to be other than Nystatin.

In another aspect, the agent is selected from aspirin, pentoxifylline, and clopidogrel bisulfate, or other angiogenic, or a rheologic active agent.

The present disclosure provides a method of treating a site of injury on a subject comprising topically administering a poloxamer to the subject in an amount effective to improve blood flow at the site of injury. In one example, the blood flow is microvascular blood flow.

Depending on such things as the type of formulation being prepared, the location to which it is to be applied, and the type of injury, disease, or disorder being treated, other agents can be added to the formulation. For example, other additives may include, a moisturizer, a humectant, a demulcent, oil, water, an emulsifier, a thickener, a thinner, an additional surface active agent, a fragrance, a preservative, an antioxidant, a hydrotropic agent, a chelating agent, a vitamin, a mineral, a permeation enhancer, a cosmetic adjuvant, a bleaching agent, a depigmentation agent, a foaming agent, a conditioner, a viscosifier, a buffering agent, and a sunscreen.

In one example, the microvasculature has a diameter ranging from about 5 µm to about 100 µm. In another example, the vessels have a diameter from about 10 µm to about 50 µm. Vessels encompassed by the treatment of the present disclosure include capillaries, arterioles, and venules.

The present disclosure also provides a method of treating a site of injury on a subject comprising topically administering a poloxamer to the patient in an amount effective to reduce inflammation at the site of injury.

The present disclosure further provides administering cells to a site of injury, disease, or disorder being treated. In one aspect, the cells are part of the composition being administered, however, the disclosure further provides for applying the cells separately. The cells encompassed by the present disclosure include stem cells, pluripotent stem cells, committed stem cells, embryonic stem cells, adult stem cells, bone marrow stem cells, adipose stem cells, umbilical cord stem cells, dura mater stem cells, precursor cells, differentiated cells, osteoblasts, myoblasts, neuroblasts, fibroblasts, glioblasts, germ cells, hepatocytes, chondrocytes, keratinocytes, melanocytes, smooth muscle cells, cardiac muscle cells, connective tissue cells, glial cells, epithelial cells, endothelial cells, hormone-secreting cells, cells of the immune system, normal cells, Schwann cells, and neurons. In some examples, it is unnecessary to pre-select the type of stem cell that is to be used, because many types of stem cells can be induced to differentiate in an organ specific pattern once delivered to a given organ or tissue. In one example, at least two different cells are used.

In the present disclosure , the pharmaceutical composition which comprises at least on surface active copolymer is useful as a wound cleanser. In one example, it is useful as a skin cleanser.

The present disclosure provides methods for identifying compounds which are useful for treating decreased blood flow associated with an injury, disease, or condition, said method comprising contacting a small bowel preparation for measuring blood flow in mesenteric vessels as described herein with a test compound, measuring the level of blood flow in the small bowel preparation with the level of blood flow in an otherwise identical small bowel preparation not treated with the test compound, wherein an increase in blood flow in the preparation treated with the compound compared to the flow in the preparation not treated with the compound is an indication that the test compound increases blood flow. In one example, each group is submitted to an injury, such as a thermal injury, prior to administration of the test compound.

The present disclosure further provides kits for administering pharmaceutical compositions of the invention to subjects in need thereof.

### BRIEF DESCRIPTION OF THE DRAWINGS

The foregoing summary, as well as the following detailed description, will be better understood when read in conjunction with the appended drawings. In the drawings:
**Figure 1** represents an image of a photomicrograph of exposed rat ileum and mesentery on the microscope platform.
**Figure 2** represents an image of a photomicrograph of a close-up view of the mesenteric area with identified areas of microvasculature.
**Figure 3** represents an image of a photomicrograph of mesenteric vessels at 280× magnification. Note the overlying mapping of each vessel segment length with unique identification numbers.

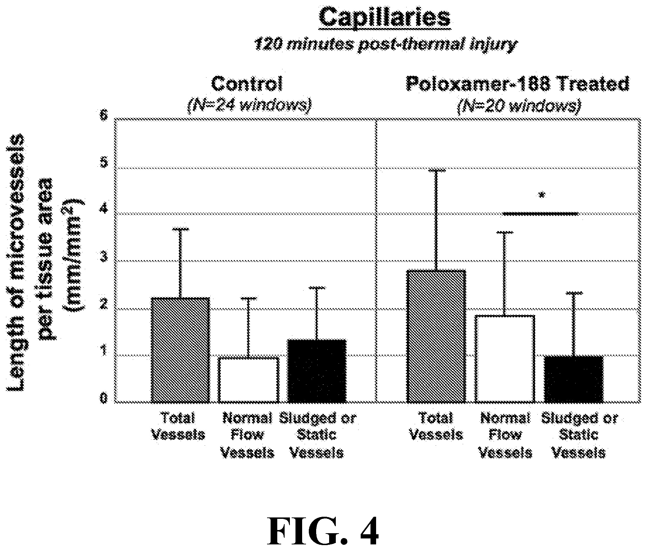

**Figure 4****,** comprising left and right panels, graphically depicts capillaries compared between control and poloxamer-188 treatment 120 minutes after thermal injury using Student t test. Control experiment (N = 24 windows) shows total length of vessel per tissue area of 2.2 mm/mm². Normal flow is noted in 0.9 mm/mm2 versus 1.3 mm/mm² with sludging or static flow (±SD). No significance was noted between these 2 values. The poloxamer-188-treated vessels (N = 20 windows) demonstrate a total of 2.8 mm/mm² with normal flow in 1.8 mm/mm² versus 1.0 mm/mm² with sludging or static flow (±SD, *P = 0.096).

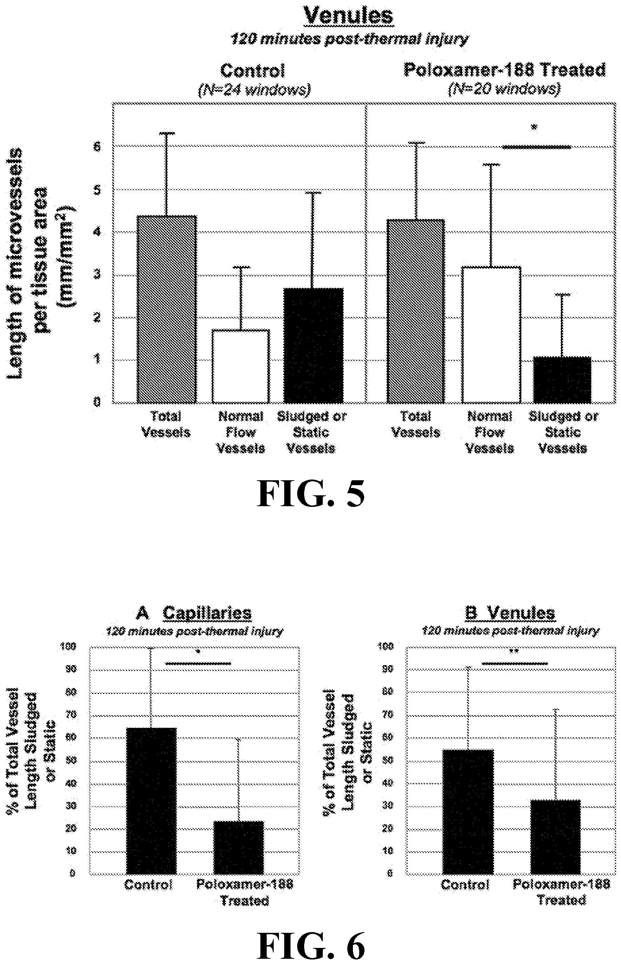

**Figure 5****,** comprising left and right panels, graphically depicts venules compared between control and poloxamer-188 treatment 120 minutes after thermal injury using Student t test. Control experiment (N = 24 windows) shows a total length of vessel per tissue area of 4.3 mm/mm2; normal flow is noted in 1.7 mm/mm2 versus 2.6 mm/mm² with sludging or static flow (±SD). No significance was noted between these 2 values. The poloxamer-188-treated vessels (N = 20 windows) demonstrate a total of 4.3 mm/mm². There was normal flow in 3.2 mm/mm² versus 1.1 mm/mm2 with sludging or static flow (±SD, *P = 0.008).
**Figure 6****,** comprising Figures 6A (left panel) and 6B (right panel), graphically depicts a comparison between control (N = 24 windows) and poloxamer-188-treated (N = 20 windows) microvessels as a percentage with abnormal flow (i.e., sludging or stasis) 120 minutes after thermal injury using Student t test. **6A:**
   Abnormal flow in 62% of control capillaries versus 23% of poloxamer-188-treated capillaries (±SD, *P = 0.002). **6B:** Abnormal flow in 54% of control venules versus 32% ofpoloxamer-188-treated capillaries (±SD, **P = 0.056).

### DETAILED DESCRIPTION OF THE INVENTION

### Abbreviations and Acronyms

ASC- adipose tissue-derived stem cell
ECM- extracellular matrix
ES- embryonic stem cell
FACS - fluorescent activated cell sorting
FBS- fetal bovine serum
FGF- fibroblast growth factor
gf- growth factor
HSC- hematopoietic stem cell
IL-1β- interleukin-1 beta
PDGF- platelet-derived growth factor
PLA- processed lipoaspirate cells
SCGF-β- stem cell growth factor-β
SFM- serum-free medium
TNFα- tumor necrosis factor alpha
VEGF- Vascular endothelial growth factor

In the present disclosure and in claiming the invention, the following terminology will be used in accordance with the definitions set forth below. Unless defined otherwise, all technical and scientific terms used herein have the same meaning as commonly understood by one of ordinary skill in the art to which this invention belongs. As used herein, each of the following terms has the meaning associated with it in this section. Specific and preferred values listed below for radicals, substituents, and ranges are for illustration only; they do not exclude other defined values or other values within defined ranges for the radicals and substituents.

As used herein, the singular forms "a," "an," and "the" include plural reference unless the context clearly dictates otherwise.

The term "about," as used herein, means approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 10%. In one aspect, the term "about" means plus or minus 20% of the numerical value of the number with which it is being used. Therefore, about 50% means in the range of 45%-55%. Numerical ranges recited herein by endpoints include all numbers and fractions subsumed within that range (e.g. 1 to 5 includes 1, 1.5, 2, 2.75, 3, 3.90, 4, and 5). It is also to be understood that all numbers and fractions thereof are presumed to be modified by the term "about."

The terms "additional therapeutically active compound" or "additional therapeutic agent", as used in the context of the present disclosure, refers to the use or administration of a compound for an additional therapeutic use for a particular injury, disease, or disorder being treated. Such a compound, for example, could include one being used to treat an unrelated disease or disorder, or a disease or disorder which may not be responsive to the primary treatment for the injury, disease or disorder being treated. Disease and disorders being treated by the additional therapeutically active agent include, for example, hypertension and diabetes. The additional compounds may also be used to treat symptoms associated with the injury, disease or disorder, including pain and inflammation.

Adipose-derived stem cells (ASC) or "adipose-derived stromal cells" refer to cells that originate from adipose tissue. By "adipose" is meant any fat tissue. The adipose tissue may be brown or white adipose tissue, derived from subcutaneous, omental/visceral, mammary, gonadal, or other adipose tissue site. Preferably, the adipose is subcutaneous white adipose tissue. Such cells may comprise a primary cell culture or an immortalized cell line. The adipose tissue may be from any organism having fat tissue. Preferably, the adipose tissue is mammalian, more preferably, the adipose tissue is human. A convenient source of adipose tissue is from liposuction surgery, however, the source of adipose tissue or the method of isolation of adipose tissue is not critical to the disclosure.

As use herein, the terms "administration of" and or "administering" a compound should be understood to mean providing a compound of the disclosure or a prodrug of a compound of the disclosure to a subject in need of treatment.

The term "adult" as used herein, is meant to refer to any non-embryonic or non-juvenile subject. For example the term "adult adipose tissue stem cell," refers to an adipose stem cell, other than that obtained from an embryo or juvenile subject.

Cells or tissue are "affected" by an injury, disease or disorder if the cells or tissue have an altered phenotype relative to the same cells or tissue in a subject not afflicted with the injury, disease, condition, or disorder.

As used herein, an "agonist" is a composition of matter that, when administered to a mammal such as a human, enhances or extends a biological activity of interest. Such effect may be direct or indirect.

A disease, condition, or disorder is "alleviated" if the severity of a symptom of the disease or disorder, the frequency with which such a symptom is experienced by a patient, or both, are reduced.

As used herein, "alleviating an injury, disease or disorder symptom," means reducing the frequency or severity of the symptom.

As used herein, an "analog" of a chemical compound is a compound that, by way of example, resembles another in structure but is not necessarily an isomer (e.g., 5-fluorouracil is an analog of thymine).

An "antagonist" is a composition of matter that when administered to a mammal such as a human, inhibits or impedes a biological activity attributable to the level or presence of an endogenous compound in the mammal. Such effect may be direct or indirect.

The term "antimicrobial agents" as used herein refers to any naturally-occurring, synthetic, or semi-synthetic compound or composition or mixture thereof, which is safe for human or animal use as practiced in the methods described herein, and is effective in killing or substantially inhibiting the growth of microbes. "Antimicrobial" as used herein, includes antibacterial, antifungal, and antiviral agents.

"Antiviral agent," as used herein means a composition of matter which, when delivered to a cell, is capable of preventing replication of a virus in the cell, preventing infection of the cell by a virus, or reversing a physiological effect of infection of the cell by a virus. Antiviral agents are well known and described in the literature. By way of example, AZT (zidovudine, Retrovir® Glaxo Wellcome Inc., Research Triangle Park, NC) is an antiviral agent which is thought to prevent replication of HIV in human cells.

The term "associated with an injury, disease, or disorder" means that, in the context of the present disclosure the decreased blood flow being treated, or prevented, occurs as a result of the injury, disease, or disorder, or that it may contribute to the injury, disease, or disorder.

The term "binding" refers to the adherence of molecules to one another, such as enzymes to substrates, ligands to receptors, antibodies to antigens, DNA binding domains of proteins to DNA, and DNA or RNA strands to complementary strands.

"Binding partner," as used herein, refers to a molecule capable of binding to another molecule.

The term "biocompatible," as used herein, refers to a material that does not elicit a substantial detrimental response in the host.

The term "biodegradable," as used herein, means capable of being biologically decomposed. A biodegradable material differs from a non-biodegradable material in that a biodegradable material can be biologically decomposed into units which may be either removed from the biological system and/or chemically incorporated into the biological system.

The term "biological sample," as used herein, refers to samples obtained from a living organism, including skin, hair, tissue, blood, plasma, cells, sweat, and urine.

The term "bioresorbable," as used herein, refers to the ability of a material to be resorbed in vivo. "Full" resorption means that no significant extracellular fragments remain. The resorption process involves elimination of the original implant materials through the action of body fluids, enzymes, or cells. Resorbed calcium carbonate may, for example, be redeposited as bone mineral, or by being otherwise re-utilized within the body, or excreted. "Strongly bioresorbable," as the term is used herein, means that at least 80% of the total mass of material implanted is resorbed within one year.

As used herein "burn" or "burns" refer to any detectable injury to tissue caused by energy applied to the tissue. The terms "burn" or "burns" further refer to any burning, or charring of the tissue, including thermal bums caused by contact with flames, hot liquids, hot surfaces, and other sources of high heat as well as steam, chemical bums, radiation, and electrical bums. First degree bums show redness; second degree bums show vesication; third degree bums show necrosis through the entire skin. Bums of the first and second degree are partial-thickness bums, those of the third degree are full-thickness bums.

The term "clearance," as used herein refers to the physiological process of removing a compound or molecule, such as by diffusion, exfoliation, removal via the bloodstream, and excretion in urine, or via sweat or other fluid.

A "compound," as used herein, refers to any type of substance or agent that is commonly considered a drug, or a candidate for use as a drug, as well as combinations and mixtures of the above.

A "control" subject is a subject having the same characteristics as a test subject, such as a similar type of dependence, etc. The control subject may, for example, be examined at precisely or nearly the same time the test subject is being treated or examined. The control subject may also, for example, be examined at a time distant from the time at which the test subject is examined, and the results of the examination of the control subject may be recorded so that the recorded results may be compared with results obtained by examination of a test subject.

A "test" subject is a subject being treated.

"Cytokine," as used herein, refers to intercellular signaling molecules, the best known of which are involved in the regulation of mammalian somatic cells. A number of families of cytokines, both growth promoting and growth inhibitory in their effects, have been characterized including, for example, interleukins, interferons, and transforming growth factors. A number of other cytokines are known to those of skill in the art. The sources, characteristics, targets and effector activities of these cytokines have been described.

The term "decreased blood flow", as used herein, refers to a decrease in blood flow at a site of injury, disease, or disorder, and includes a decrease in flow rate, an increase in stasis, and an increase in sludging in the vessels.

As used herein, a "derivative" of a compound refers to a chemical compound that may be produced from another compound of similar structure in one or more steps, as in replacement of H by an alkyl, acyl, or amino group.

The use of the word "detect" and its grammatical variants is meant to refer to measurement of the species without quantification, whereas use of the word "determine" or "measure" with their grammatical variants are meant to refer to measurement of the species with quantification. The terms "detect" and "identify" are used interchangeably herein.

As used herein, a "detectable marker" or a "reporter molecule" is an atom or a molecule that permits the specific detection of a compound comprising the marker in the presence of similar compounds without a marker. Detectable markers or reporter molecules includes radioactive isotopes, antigenic determinants, enzymes, nucleic acids available for hybridization, chromophores, fluorophores, chemiluminescent molecules, electrochemically detectable molecules, and molecules that provide for altered fluorescence polarization or altered light scattering.

A "disease" is a state of health of an animal wherein the animal cannot maintain homeostasis, and wherein if the disease is not ameliorated then the animal's health continues to deteriorate. As used herein, normal aging is included as a disease.

A "disorder" in an animal is a state of health in which the animal is able to maintain homeostasis, but in which the animal's state of health is less favorable than it would be in the absence of the disorder. Left untreated, a disorder does not necessarily cause a further decrease in the animal's state of health.

As used herein, an "effective amount" means an amount sufficient to produce a selected effect, such as alleviating symptoms of a disease or disorder. In the context of administering compounds in the form of a combination, such as multiple compounds, the amount of each compound, when administered in combination with another compound(s), may be different from when that compound is administered alone. Thus, an effective amount of a combination of compounds refers collectively to the combination as a whole, although the actual amounts of each compound may vary. The term "more effective" means that the selected effect is alleviated to a greater extent by one treatment relative to the second treatment to which it is being compared.

As used herein, a "functional" molecule is a molecule in a form in which it exhibits a property or activity by which it is characterized. A functional enzyme, for example, is one that exhibits the characteristic catalytic activity by which the enzyme is characterized.

"Graft" refers to any free (unattached) cell, tissue, or organ for transplantation.

"Allograft" refers to a transplanted cell, tissue, or organ derived from a different animal of the same species.

"Xenograft" refers to a transplanted cell, tissue, or organ derived from an animal of a different species.

The term "growth factor" as used herein means a bioactive molecule that promotes the proliferation of a cell or tissue. Growth factors useful in the present disclosure include transforming growth factor-alpha (TGF-α), transforming growth factor-beta (TGF-β), platelet-derived growth factors including the AA, AB and BB isoforms (PDGF), fibroblast growth factors (FGF), including FGF acidic isoforms 1 and 2, FGF basic form 2, and FGF 4, 8, 9 and 10, nerve growth factors (NGF) including NGF 2.5s, NGF 7.0s and beta NGF and neurotrophins, brain derived neurotrophic factor, cartilage derived factor, bone growth factors (BGF), basic fibroblast growth factor, insulin-like growth factor (IGF), vascular endothelial growth factor (VEGF), EG-VEGF, VEGF-related protein, Bv8, VEGF-E, granulocyte colony stimulating factor (G-CSF), insulin like growth factor (IGF) I and II, hepatocyte growth factor, glial neurotrophic growth factor, stem cell factor (SCF), keratinocyte growth factor (KGF), skeletal growth factor, bone matrix derived growth factors, and bone derived growth factors and mixtures thereof. Some growth factors may also promote differentiation of a cell or tissue. TGF, for example, may promote growth and/or differentiation of a cell or tissue.

The term "improved blood flow," as used herein, refers to increased blood flow in a subject being treated according to the methods of the present disclosure compared with the flow in a subject with an otherwise identical injury or condition not being treated according to the methods of the present disclosure. Improved flow is determined by methods such as those described herein and can include less stasis, less sludging, or a combination of both, in the subject being treated compared with the untreated subject.

The term "inhibit," as used herein, refers to the ability of a compound, agent, or method to reduce or impede a described function, level, activity, rate, etc., based on the context in which the term "inhibit" is used. Preferably, inhibition is by at least 10%, more preferably by at least 25%, even more preferably by at least 50%, and most preferably, the function is inhibited by at least 75%. The term "inhibit" is used interchangeably with "reduce" and "block."

"Inhibiting decreased blood flow" as described herein, refers to any method or technique which inhibits the decrease in blood flow or associated changes in blood flow following injury, or where decreased blood flow is associated with a disease or disorder, particularly thermal injury. Methods of measuring blood flow are described herein. Inhibition can be direct or indirect.

As used herein "injecting or applying" includes administration of a compound of the disclosure by any number of routes and means including topical, oral, buccal, intravenous, intramuscular, intra arterial, intramedullary, intrathecal, intraventricular, transdermal, subcutaneous, intraperitoneal, intranasal, enteral, topical, sublingual, vaginal, ophthalmic, pulmonary, or rectal means.

As used herein, "injury" generally refers to damage, harm, or hurt; usually applied to damage inflicted on the body by an external force.

As used herein, an "instructional material" includes a publication, a recording, a diagram, or any other medium of expression which can be used to communicate the usefulness of a compound of the disclosure in the kit for effecting alleviation of the various diseases or disorders recited herein. Optionally, or alternately, the instructional material may describe one or more methods of alleviating the diseases or disorders in a subject. The instructional material of the kit may, for example, be affixed to a container which contains the identified compound disclosure or be shipped together with a container which contains the identified compound. Alternatively, the instructional material may be shipped separately from the container with the intention that the instructional material and the compound be used cooperatively by the recipient.

As used herein, a "ligand" is a compound that specifically binds to a target compound or molecule. A ligand "specifically binds to" or "is specifically reactive with" a compound when the ligand functions in a binding reaction which is determinative of the presence of the compound in a sample of heterogeneous compounds.

As used herein, "parenteral administration" of a pharmaceutical composition includes any route of administration characterized by physical breaching of a tissue of a subject and administration of the pharmaceutical composition through the breach in the tissue. Parenteral administration thus includes administration of a pharmaceutical composition by injection of the composition, by application of the composition through a surgical incision, by application of the composition through a tissue-penetrating non-surgical wound. In particular, parenteral administration is contemplated to include subcutaneous, intraperitoneal, intramuscular, intrasternal injection, and kidney dialytic infusion techniques.

"Permeation enhancement" and "permeation enhancers" as used herein relate to the process and added materials which bring about an increase in the permeability of skin to a poorly skin permeating pharmacologically active agent, i.e., so as to increase the rate at which the drug permeates through the skin and enters the bloodstream. "Permeation enhancer" is used interchangeably with "penetration enhancer".

The term "pharmaceutical composition" shall mean a composition comprising at least one active ingredient, whereby the composition is amenable to investigation for a specified, efficacious outcome in a mammal (for example, without limitation, a human). Those of ordinary skill in the art will understand and appreciate the techniques appropriate for determining whether an active ingredient has a desired efficacious outcome based upon the needs of the artisan.

As used herein, the term "pharmaceutically-acceptable carrier" means a chemical composition with which an appropriate compound or derivative can be combined and which, following the combination, can be used to administer the appropriate compound to a subject.

As used herein, the term "physiologically acceptable" ester or salt means an ester or salt form of the active ingredient which is compatible with any other ingredients of the pharmaceutical composition, which is not deleterious to the subject to which the composition is to be administered.

The term "prevent," as used herein, means to stop something from happening, or taking advance measures against something possible or probable from happening. In the context of medicine, "prevention" generally refers to action taken to decrease the chance of getting a disease or condition.

A "prophylactic" treatment is a treatment administered to a subject who does not exhibit signs of a disease or injury or exhibits only early signs of the disease or injury for the purpose of decreasing the risk of developing pathology associated with the disease or injury.

As used herein, the term "purified" and like terms relate to an enrichment of a molecule or compound relative to other components normally associated with the molecule or compound in a native environment. The term "purified" does not necessarily indicate that complete purity of the particular molecule has been achieved during the process. A "highly purified" compound as used herein refers to a compound that is greater than 90% pure.

### "Reduce"- see "inhibit."

The term "regulate" refers to either stimulating or inhibiting a function or activity of interest.

A "receptor" is a compound or molecule that specifically binds to a ligand. A "sample," as used herein, refers to a biological sample from a subject, including normal tissue samples, diseased tissue samples, biopsies, blood, saliva, feces, semen, tears, and urine. A sample can also be any other source of material obtained from a subject which contains cells, tissues, or fluid of interest.

The term "skin," as used herein, refers to the commonly used definition of skin, e.g., the epidermis and dermis, and the cells, glands, mucosa, and connective tissue which comprise the skin.

By the term "specifically binds," as used herein, is meant a molecule which recognizes and binds a specific molecule, but does not substantially recognize or bind other molecules in a sample, or it means binding between two or more molecules as in part of a cellular regulatory process, where said molecules do not substantially recognize or bind other molecules in a sample.

The term "standard," as used herein, refers to something used for comparison. For example, it can be a known standard agent or compound which is administered and used for comparing results when administering a test compound, or it can be a standard parameter or function which is measured to obtain a control value when measuring an effect of an agent or compound on a parameter or function. "Standard" can also refer to an "internal standard", such as an agent or compound which is added at known amounts to a sample and which is useful in determining such things as purification or recovery rates when a sample is processed or subjected to purification or extraction procedures before a marker of interest is measured. Internal standards are often a purified marker of interest which has been labeled, such as with a radioactive isotope, allowing it to be distinguished from an endogenous substance in a sample.

A "subject" of diagnosis or treatment is a mammal, including a human.

As used herein, a "subject in need thereof" is a patient, animal, mammal, or human, who will benefit from the method of this disclosure.

A "surface active agent" or "surfactant" is a substance that has the ability to reduce the surface tension of materials and enable penetration into and through materials.

The term "symptom," as used herein, refers to any morbid phenomenon or departure from the normal in structure, function, or sensation, experienced by the patient and indicative of disease. In contrast, a sign is objective evidence of disease. For example, a bloody nose is a sign. It is evident to the patient, doctor, nurse and other observers.

A "therapeutic" treatment is a treatment administered to a subject who exhibits signs of pathology for the purpose of diminishing or eliminating those signs.

A "therapeutically effective amount" of a compound is that amount of compound which is sufficient to provide a beneficial effect to the subject to which the compound is administered.

The term "thermal injury" is used interchangeably with "thermal burn" herein.

"Tissue" means (1) a group of similar cells united to perform a specific function; (2) a part of an organism consisting of an aggregate of cells having a similar structure and function; or (3) a grouping of cells that are similarly characterized by their structure and function, such as muscle or nerve tissue.

The term "tissue injury-associated decreased blood flow", as used herein, refers to the decrease in blood flow which occurs following an injury, such as a thermal injury, to a tissue. The decrease in blood flow includes decreased volume, rate, stasis, or sludging. One of ordinary skill in the art will appreciate that there are multiple parameters which can be used as measures or signs of decreased blood flow, as well as multiple techniques to determine decreased blood flow.

The term "topical application," as used herein, refers to administration to a surface, such as the skin. This term is used interchangeably with "cutaneous application" in the case of skin. A "topical application" is a "direct application".

By "transdermal" delivery is meant delivery by passage of a drug through the skin or mucosal tissue and into the bloodstream. Transdermal also refers to the skin as a portal for the administration of drugs or compounds by topical application of the drug or compound thereto. "Transdermal" is used interchangeably with "percutaneous."

As used herein, the term "treating" may include prophylaxis of the specific injury, disease, disorder, or condition, or alleviation of the symptoms associated with a specific injury, disease, disorder, or condition and/or preventing or eliminating said symptoms. A "prophylactic" treatment is a treatment administered to a subject who does not exhibit signs of a disease or exhibits only early signs of the disease for the purpose of decreasing the risk of developing pathology associated with the disease. "Treating" is used interchangeably with "treatment" herein.

As used herein "wound" or "wounds" may refer to any detectable break in the tissues of the body, such as injury to skin or to an injury or damage, or to a damaged site associated with a disease or disorder. Although the terms "wound" and "injury" are not always defined exactly the same way, the use of one term herein, such as "injury", is not meant to exclude the meaning of the other term.

### Chemical Definitions

As used herein, the term "halogen" or "halo" includes bromo, chloro, fluoro, and iodo.

The term "haloalkyl" as used herein refers to an alkyl radical bearing at least one halogen substituent, for example, chloromethyl, fluoroethyl or trifluoromethyl

The term "C₁-Cₙ alkyl" wherein n is an integer, as used herein, represents a branched or linear alkyl group having from one to the specified number of carbon atoms. Typically, C₁-C₆ alkyl groups include methyl, ethyl, n-propyl, iso-propyl, butyl, iso-butyl, sec-butyl, tert-butyl, pentyl and hexyl. The term "C₂-Cₙ alkenyl" wherein n is an integer, as used herein, represents an olefinically unsaturated branched or linear group having from two to the specified number of carbon atoms and at least one double bond. Examples of such groups include 1-propenyl, 2-propenyl, 1,3-butadienyl, 1-butenyl, hexenyl and pentenyl. The term "C₂-Cₙ alkynyl" wherein n is an integer refers to an unsaturated branched or linear group having from two to the specified number of carbon atoms and at least one triple bond. Examples of such groups include 1-propynyl, 2-propynyl, 1-butynyl, 2-butynyl and 1-pentynyl. The term "C₃-Cₙ cycloalkyl" wherein n = 8, represents cyclopropyl, cyclobutyl, cyclopentyl, cyclohexyl, cycloheptyl, and cyclooctyl.

As used herein the term "aryl" refers to an optionally substituted mono- or bicyclic carbocyclic ring system having one or two aromatic rings including phenyl, benzyl, naphthyl, tetrahydronaphthyl, indanyl and indenyl. "Optionally substituted aryl" includes aryl compounds having from zero to four substituents, and "substituted aryl" includes aryl compounds having one or more substituents. The term (C₅-C₈ alkyl)aryl refers to any aryl group which is attached to the parent moiety via the alkyl group.

The term "bicyclic" represents either an unsaturated or saturated stable 7- to 12-membered bridged or fused bicyclic carbon ring. The bicyclic ring may be attached at any carbon atom which affords a stable structure. The term includes naphthyl, dicyclo hexyl and dicyclohexenyl. The term "heterocyclic group" refers to an optionally substituted mono- or bicyclic carbocyclic ring system containing from one to three heteroatoms wherein the heteroatoms are selected from the group consisting of oxygen, sulfur, and nitrogen.

As used herein the term "heteroaryl" refers to an optionally substituted mono- or bicyclic carbocyclic ring system having one or two aromatic rings containing from one to three heteroatoms and includes furyl, thienyl and pyridyl. A "meroxapol" is polyoxypropylene-polyoxyethylene block copolymer with the general formula HO(C₃H₆O)ₐ(C₂H₄O)_{b}(C₃H₆O)ₐH. It is available in different grades. Each meroxapol name is followed by a code number according to the average numerical values of the respective monomers units denoted by "a" and "b".

As used herein, the term "optionally substituted" refers to from zero to four substituents, wherein the substituents are each independently selected. Each of the independently selected substituents may be the same or different than other substituents.

A "poloxamer" is a nonionic polyoxyethylene-polyoxypropylene block copolymer with the general formula HO(C₂H₄O)ₐ(C₃H₆O)_{b}(C₂H₄O)ₐH. It is available in different grades, which vary from liquids to solids. Each poloxamer name is followed by a code number according to the average numerical values of the respective monomers units denoted by "a" and "b".

A "poloxamine" is a polyoxyethylen-polyoxypropylene block copolymer of ethylene diamine with the general formula [HO(C₂H₄O)ₐ(C₃H₆O)_{b}C₃H₆]₂NCH₂CH₂N-[C₃H₆(OC₃H₆)_{b}(OC₂H₄)ₐOH]₂. It is available in different grades. Each poloxamine name is followed by a code number according to the average numerical values of the respective monomers units denoted by "a" and "b".

The compounds described herein contain one or more asymmetric centers in the molecule. In accordance herewith a structure that does not designate the stereochemistry is to be understood as embracing all the various optical isomers, as well as racemic mixtures thereof.

The compounds described herein may exist in tautomeric forms and includes both mixtures and separate individual tautomers. For example the following structure: 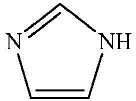 is understood to represent a mixture of the structures:

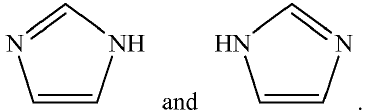

### Specific Description

The present disclosure relates to the use of surface active copolymers, including poloxamers, to inhibit the decreased blood flow, including stasis and sludging, which results from injuries such as thermal injury or decreased blood flow associated with diseases and disorders. More specifically, the present disclosure relates to topical application of a composition comprising at least one poloxamer or other surface active copolymer to an injured or diseased site where there is decreased blood flow, or to a site to prevent decreased blood flow, such as before a surgical procedure. In one aspect, the decreased blood flow includes decreased flow in microvasculature, including capillaries, venules, and arterioles. The formulations of the disclosure may comprise additional therapeutic agents, for example, antimicrobial agents to prevent infection, growth factors or hormones to enhance healing, drugs to treat inflammation, or anesthetics to decrease pain.

According to one embodiment, a poloxamer such as poloxamer-188 (Pluronic F68) is the base compound of the composition of the invention. A poloxamer has the special ability to thicken at higher temperatures (such as body temperature) and liquefy at cooler temperatures (cool rinse water for example). The thickness, or viscosity, varies depending on the amount or concentration of surface active copolymer used. These properties enable it to remain resident at tissue surfaces at body temperature but also enable it to be easily removed away with cool water. Preferred surface active copolymers are those which are biocompatible. In addition, dilutions of surface active copolymers such as Poloxamer-188 (Pluronic F68) are very biocompatible.

### Injuries, Wounds, Diseases, and Disorders

A subject having a site of injury or wound, or in some cases a disease or disorder, may be susceptible to decreased blood flow at that site and therefore be in need of treatment. In one aspect, the decreased blood flow is in microvessels. These conditions may typically arise from many types of injury including surgery and trauma to the skin and/or exposed soft tissue, resulting in an inflammatory reaction and decreased blood flow, particularly in the microvasculature. The types of injuries, disease, and disorders encompassed by the methods of the present disclosure therefore include, bums, chronic wounds, and surgical procedures such as microvascular surgery, skin flaps and skin grafts, and tissue injury resulting from, for example, a burn, scrape, cut, incision, laceration, ulcer, body piercing, bite wound, trauma, stab wound, gunshot wound, surgical wound, stretch injury, crush wound, compression wound, fracture, sprain, strain, stroke, infarction, aneurysm, herniation, ischemia, fistula, dislocation, radiation, cell, tissue or organ grafting, injuries sustained during medical procedures, or cancer.

Such injuries include skin injury, muscle injury, brain injury, eye injury, or spinal cord injury. Tissue injury can include joint injury, back injury, heart injury, vascular system injury, soft tissue injury, cartilage injury, lymphatic system injury, tendon injury, ligament injury, or abdominal injury.

The injuries that are contemplated to be treated by use of a composition of the present invention include, for example, any denuded area without skin or mucosa that is due to trauma such as a burn, a surgical trauma, an abrasion, a malignancy, an infection, or an allergic reaction. It is believed that the use of the composition of present invention will result in an improved cosmetic and functional outcome for the subjects being treated.

The invention can be used to treat all types of thermal injuries and bums. These include acute conditions such as thermal bums, chemical bums, radiation bums, bums caused by excess exposure to ultraviolet radiation such as sunburn, as well as by the chronic wounds associated with some of these conditions.

Bums include first degree bums which may cause skin manifestations such as reddening, pain, and/or mild swelling. One non-limiting example of first degree burn is a sun burn. Bums further refers to second-degree bums involving the first two layers of skin. Signs of second degree burning include, among other things, deep reddening of the skin, blisters, pain, glossy appearance from leaking fluid, and possible skin loss. Bums further refers to third-degree bums which penetrate the entire thickness of the skin and may destroy tissue. Signs of third degree burning include, among other things, loss of skin, dry skin, leathery skin, charred skin having a mottled appearance, and combinations thereof. In some cases, skin with a third degree burn may be painless.

It is contemplated that the compositions of the present invention will benefit, for example, subjects suffering from vesicant bums and thermal bums, including first degree bums, second degree bums and third degree bums, as well as esophageal bums and erosions. For example, after cutaneous burn injury, an area surrounding the wound is the site of a pronounced inflammatory response with an associated reduced blood flow. This "zone of stasis" undergoes progressive necrosis within 24-48 hours resulting in an expansion of the burn wound characterized by decreased microvascular blood flow.

Injuries encompassed herein further include acute and chronic wounds. Chronic wounds are wounds characterized by non-healing skin wounds and include chronic venous ulcers, diabetic ulcers, arterial ulcers, pressure ulcers (e.g., decubitus ulcers), radiation ulcers, traumatic wounds, and open, complicated non-healing wounds. Wounds further refers to cuts and scrapes known as open wounds, as well as others, such as deep bruises, or closed wounds. Non-limiting examples of wounds suitable for treatment in accordance with the present disclosure include abrasions such as those caused by: scraping the outer layer of skin; incisions such as those caused by sharp edges, knives, metal edges, broken glass or other sharp object; lacerations or jagged, irregular cuts or tears of the skin; punctures such as those caused by an object piercing the skin layers and creating a small hole; and/or bums. Additional non-limiting wounds suitable for treatment in accordance with the present disclosure include puncture wounds, gaping wounds, wounds having fatty layers, tissue or muscle exposed, wounds having one or more foreign bodies therein, wounds causing severe pain, wounds having blood flowing there from, or any wound that causes numbness or loss of movement below the wound.

Other non-limiting examples of wounds suitable for treatment in accordance with the present disclosure include animal bites, arterial disease, insect stings and bites, bone infections, compromised skin/muscle grafts, gangrene, skin tears or lacerations, surgical incisions, including slow or non-healing surgical wounds, and post-operation infections. It is understood, that the listed wounds are non-limiting and that only a portion of wounds suitable for treatment in accordance with the present disclosure are listed herein.

It is also contemplated that the composition of the present invention will benefit subjects with chronic skin ulcers, including but limited to decubitus ulcers, venous stasis ulcers, arterial insufficiency ulcers, and diabetic foot ulcers.

### Compositions and Formulations of the Base Surface Active Copolymer

The disclosure encompasses the preparation and use of pharmaceutical compositions comprising as an active ingredient a compound useful for treatment of decreased blood flow associated with injuries and diseases disclosed herein. Such a pharmaceutical composition may consist of the active ingredient alone, in a form suitable for administration to a subject, or the pharmaceutical composition may comprise the active ingredient and one or more pharmaceutically acceptable carriers, one or more additional ingredients, or some combination of these. The active ingredient may be present in the pharmaceutical composition in the form of a physiologically acceptable ester or salt, such as in combination with a physiologically acceptable cation or anion, as is well known in the art. The present invention further contemplates the use of more than one active ingredient.

The disclosure is not limited to the use of poloxamer-188 (Pluronic F68) but may include the use an additional surface active copolymer, examples of which include other poloxamers, meroxapols, and poloxamines. Examples of poloxamers include poloxamer-101, -105, -105 benzoate, -108, -122, -123, -124, -181, -182, - 182 dibenzoate, -183, -184, -185, -188, -212, -215, -217, -231, -234, -235, -237,-238, -282, -284, -288, -331, -333, -334, -335, -338, -401, -402, -403, and -407. Examples of meroxapols include meroxapol 105, 108, 171, 172, 174, 178, 251, 252, 254, 258, 311, 312, and 314. Examples of poloxamines include poloxamine 304, 504, 701, 702, 704, 707, 901, 904, 908, 1101, 1102, 1104, 1301, 1302, 1304, 1307, 1501, 1502, 1504, and 1508.

In one embodiment, at least two different surface active copolymers are used. In one aspect, at least three different surface active copolymers are used. These combinations may include, for example, one or more poloxamers, one or more meroxapols, and one or more poloxamines.

The copolymers of the disclosure may vary in size. The copolymer size may range, for example, from an Mₙ of about 600 to about 20,000, or in another aspect from about 1,000 to about 10,000. The weight of hydrophobic groups can be from about 45-95% by weight of the copolymer. A formulation comprising at least one copolymer (*e.g*., a poloxamer, meroxapol, or poloxamine) and water can be prepared by cooling it to an appropriate temperature, or by other methods known in the art. Compositions of this type are described in U.S. Patent No. 5,635,540 (Edlich et al.).

Examples of temperature ranges for preparation include from about -20°C to about 15°C, in another aspect from about -18°C to about 8°C, and in another aspect, from about -15°C to about 5°C. These ranges also encompass about 0°F to about 60°F. One of ordinary skill in the art will understand that the temperatures of preparation can be adjusted based on various criteria, such as the surface active copolymer being used, the amount or concentration being used, the type of formulation being prepared for administration, etc.

In one example, the poloxamer base comprises 80% polyoxyethylene units and 20% polyoxypropylene units.

One of ordinary skill in the art will appreciate that the formulations, method of preparation, and amount of surface active copolymer used may vary, depending on the type or location of the site to be treated. For example, a poloxamer, such as poloxamer-188, is mixed with water at a ratio of from 1:0.8 to 1.2 w/w. This ratio can be varied. This combination may be mixed until the powder has been wetted. The mixture may then be placed in a freezer or refrigerator and cooled, preferably for at least 4 hours. While cooling, the mixture will undergo phase transition to a liquid, as demonstrated by Edlich et al. (U.S. Pat. No. 5,635,540). The mixture is then removed from the freezer and warmed to room temperature. Pharmaceutical agents such as antimicrobials and anesthetics can be added at this point, as demonstrated by Edlich et al. (U.S. Pat. No. 5,635,540).

The poloxamer base used in preparing the topical preparation of the present disclosure is a polyoxyalkylene based polymer based on ethylene oxide and propylene oxide and comprises a series of closely related block polymers that may generally be classified as polyoxyethylene-polyoxypropylene condensates terminated in primary hydroxyl groups. They are formed by the condensation of propylene oxide onto a propylene glycol nucleus followed by condensation of ethylene oxide onto both ends of the polyoxypropylene base. The polyoxyethylene hydrophilic groups on the ends of the molecule are controlled in length to constitute anywhere from 10% to 90% by weight of the final molecule.

In one embodiment, the molecular weight Mn of the poloxamer base ranges from about 600 to about 20,000. In one aspect, it ranges from about 1,000 to about 1000. In another aspect, it ranges from about 5,000 to about 8,500.

The compositions describe herein may comprise one or more co-additives (e.g., solvent such as water). In one example, the concentration of a surface active copolymer (e.g., poloxamer 188) is about 0.01 to about 99.99% w/w. In another example, it is about 1 to about 90%. In yet another example, it is about 10 to about 80%. In a further example, it is about 20% to about 70%. In another example, it is about 50%. According to the claimed invetion, it is 5%.

A formulation of the disclosure can be impregnated in a dressing material (or otherwise contained or encompassed by the dressing material). The dressing material is a pharmaceutically acceptable fabric. It can be, for example, gauze or any other type of medical fabric or material that can be used to cover a wound and/or to keep a therapeutic agent or composition in contact with a patient.

### Additional Therapeutic Agents and Ingredients

The composition of the invention can further comprise additional therapeutic additives, alone or in combination (*e.g*., 2, 3, or 4 additional additives). Examples of additional additives include : (a) antimicrobials, (b) steroids (*e.g.,* hydrocortisone, triamcinolone); (c) pain medications (*e.g.,* aspirin, an NSAID, and a local anesthetic); (d) anti-inflammatory agents; (e) growth factors; (f) cytokines; (g) hormones; and (h) combinations thereof.

In one embodiment, a formulation of the disclosure contains an antimicrobial agent. The antimicrobial agent may be provided at, for example, a standard therapeutically effective amount. A standard therapeutically effective amount is an amount that is typically used by one of ordinary skill in the art or an amount approved by a regulatory agency (*e.g.,* the FDA or its European counterpart). Antimicrobial agents useful for the disclosure include those directed against the spectrums of gram positive organisms, gram negative organisms, fungi, and viruses.

According to the topical anesthetic embodiment of the present disclosure in one aspect, suitable local anesthetic agents having a melting point of 30° to 70°C are prilocaine, tetracaine, butanilcaine, trimecaine, benzocaine, lidocaine, bupivocaine, dibucaine, mepivocaine, and etidocaine.

The present disclosure further encompasses the use of at least two anesthetics.

The local anesthetic composition may further comprise suitable additives, such a pigment, a dye, an anti-oxidant, a stabilizer or a fragrance provided that addition of such an additive does not destroy the single phase of the anesthetic composition.

In one example, the hydrated local anesthetic mixture is prepared by melting the local anesthetic with the higher melting point of the two, followed by addition of the other local anesthetic, under vigorous mechanical mixing, such as trituration or grinding. A milky viscous liquid is formed, at which point, the surfactant is added with more mechanical mixing. Mixing of the surfactant produces a milky liquid of somewhat lower viscosity. Finally, the balance of water is added under vigorous mechanical mixing. The material can then be transferred to an air tight container, after which a clear composition is obtained after about 60 minutes at room temperature.

Alternatively, the hydrated local anesthetic mixture can be prepared by first melting the lower melting local anesthetic, followed by addition of the other local anesthetic along with vigorous mechanical mixing, then addition of the surfactant and water as above. However, when the lower melting local anesthetic is melted first, the storage time needed to obtain the single phase composition, increases from about 1 hour to about 72 hours. Accordingly, the former method is preferred.

One of ordinary skill in the art will appreciate that there are multiple suitable surfactants useful for preparing the hydrated topical anesthetic of the present disclosure. For example, single-phase hydrated topical anesthetics can be prepared from anionic, cationic or non-ionic surfactants.

Several preferred embodiments include use of any therapeutic molecule including any pharmaceutical or drug. Examples of pharmaceuticals include anesthetics, hypnotics, sedatives and sleep inducers, antipsychotics, antidepressants, antiallergics, antianginals, antiarthritics, antiasthmatics, antidiabetics, antidiarrheal drugs, anticonvulsants, antigout drugs, antihistamines, antipruritics, emetics, antiemetics, antispasmodics, appetite suppressants, neuroactive substances, neurotransmitter agonists, antagonists, receptor blockers and reuptake modulators, beta-adrenergic blockers, calcium channel blockers, disulfiram and disulfiram-like drugs, muscle relaxants, analgesics, antipyretics, stimulants, anticholinesterase agents, parasympathomimetic agents, hormones, anticoagulants, antithrombotics, thrombolytics, immunoglobulins, immunosuppressants, hormone agonists/antagonists, vitamins, antimicrobial agents, antineoplastics, antacids, digestants, laxatives, cathartics, antiseptics, diuretics, disinfectants, fungicides, ectoparasiticides, antiparasitics, heavy metals, heavy metal antagonists, chelating agents, gases and vapors, alkaloids, salts, ions, autacoids, digitalis, cardiac glycosides, antiarrhythmics, antihypertensives, vasodilators, vasoconstrictors, antimuscarinics, ganglionic stimulating agents, ganglionic blocking agents, neuromuscular blocking agents, adrenergic nerve inhibitors, antioxidants, vitamins, cosmetics, anti-inflammatories, wound care products, antithrombogenic agents, antitumoral agents, antiangiogenic agents, anesthetics, antigenic agents, wound healing agents, plant extracts, growth factors, emollients, humectants, rejection/anti-rejection drugs, spermicides, conditioners, antibacterial agents, antifungal agents, antiviral agents, antibiotics, tranquilizers, cholesterol-reducing drugs, antitussives, histamine-blocking drugs, monoamine oxidase inhibitor. All substances listed by the U.S. Pharmacopeia are also included within the substances of the present invention.

A list of the types of drugs, and specific drugs within categories which are encompassed within the invention is provided below and are intended be non-limiting examples.

Antimicrobial agents include: silver sulfadiazine, Nystatin, Nystatin/triamcinolone, Bacitracin, nitrofurazone, nitrofurantoin, a polymyxin (*e.g*., Colistin, Surfactin, Polymyxin E, and Polymyxin B), doxycycline, antimicrobial peptides (*e.g*., natural and synthetic origin), Neosporin (i.e., Bacitracin, Polymyxin B, and Neomycin), Polysporin (i.e., Bacitracin and Polymyxin B). Additional antimicrobials include topical antimicrobials (i.e., antiseptics), examples of which include silver salts, iodine, benzalkonium chloride, alcohol, hydrogen peroxide, and chlorhexidine.

Analgesic: Acetaminophen; Alfentanil Hydrochloride; Aminobenzoate Potassium; Aminobenzoate Sodium; Anidoxime; Anileridine; Anileridine Hydrochloride; Anilopam Hydrochloride; Anirolac; Antipyrine; Aspirin; Benoxaprofen; Benzydamine Hydrochloride; Bicifadine Hydrochloride; Brifentanil Hydrochloride; Bromadoline Maleate; Bromfenac Sodium; Buprenorphine Hydrochloride; Butacetin; Butixirate; Butorphanol; Butorphanol Tartrate; Carbamazepine; Carbaspirin Calcium; Carbiphene Hydrochloride; Carfentanil Citrate; Ciprefadol Succinate; Ciramadol; Ciramadol Hydrochloride; Clonixeril; Clonixin; Codeine; Codeine Phosphate; Codeine Sulfate; Conorphone Hydrochloride; Cyclazocine; Dexoxadrol Hydrochloride; Dexpemedolac; Dezocine; Diflunisal; Dihydrocodeine Bitartrate; Dimefadane; Dipyrone; Doxpicomine Hydrochloride; Drinidene; Enadoline Hydrochloride; Epirizole; Ergotamine Tartrate; Ethoxazene Hydrochloride; Etofenamate; Eugenol; Fenoprofen; Fenoprofen Calcium; Fentanyl Citrate; Floctafenine; Flufenisal; Flunixin; Flunixin Meglumine; Flupirtine Maleate; Fluproquazone; Fluradoline Hydrochloride; Flurbiprofen; Hydromorphone Hydrochloride; Ibufenac; Indoprofen; Ketazocine; Ketorfanol; Ketorolac Tromethamine; Letimide Hydrochloride; Levomethadyl Acetate; Levomethadyl Acetate Hydrochloride; Levonantradol Hydrochloride; Levorphanol Tartrate; Lofemizole Hydrochloride; Lofentanil Oxalate; Lorcinadol; Lomoxicam; Magnesium Salicylate; Mefenamic Acid; Menabitan Hydrochloride; Meperidine Hydrochloride; Meptazinol Hydrochloride; Methadone Hydrochloride; Methadyl Acetate; Methopholine; Methotrimeprazine; Metkephamid Acetate; Mimbane Hydrochloride; Mirfentanil Hydrochloride; Molinazone; Morphine Sulfate; Moxazocine; Nabitan Hydrochloride; Nalbuphine Hydrochloride; Nalmexone Hydrochloride; Namoxyrate; Nantradol Hydrochloride; Naproxen; Naproxen Sodium; Naproxol; Nefopam Hydrochloride; Nexeridine Hydrochloride; Noracymethadol Hydrochloride; Ocfentanil Hydrochloride; Octazamide; Olvanil; Oxetorone Fumarate; Oxycodone; Oxycodone Hydrochloride; Oxycodone Terephthalate; Oxymorphone Hydrochloride; Pemedolac; Pentamorphone; Pentazocine; Pentazocine Hydrochloride; Pentazocine Lactate; Phenazopyridine Hydrochloride; Phenyramidol Hydrochloride; Picenadol Hydrochloride; Pinadoline; Pirfenidone; Piroxicam Olamine; Pravadoline Maleate; Prodilidine Hydrochloride; Profadol Hydrochloride; Propirarn Fumarate; Propoxyphene Hydrochloride; Propoxyphene Napsylate; Proxazole; Proxazole Citrate; Proxorphan Tartrate; Pyrroliphene Hydrochloride; Remifentanil Hydrochloride; Salcolex; Salethamide Maleate; Salicylamide; Salicylate Meglumine; Salsalate; Sodium Salicylate; Spiradoline Mesylate; Sufentanil; Sufentanil Citrate; Talmetacin; Talniflumate; Talosalate; Tazadolene Succinate; Tebufelone; Tetrydamine; Tifurac Sodium; Tilidine Hydrochloride; Tiopinac; Tonazocine Mesylate; Tramadol Hydrochloride; Trefentanil Hydrochloride; Trolamine; Veradoline Hydrochloride; Verilopam Hydrochloride; Volazocine; Xorphanol Mesylate; Xylazine Hydrochloride; Zenazocine Mesylate; Zomepirac Sodium; Zucapsaicin.

Antihypertensive: Aflyzosin Hydrochloride; Alipamide; Althiazide; Amiquinsin Hydrochloride; Amlodipine Besylate; Amlodipine Maleate; Anaritide Acetate; Atiprosin Maleate; Belfosdil; Bemitradine; Bendacalol Mesylate; Bendroflumethiazide; Benzthiazide; Betaxolol Hydrochloride; Bethanidine Sulfate; Bevantolol Hydrochloride; Biclodil Hydrochloride; Bisoprolol; Bisoprolol Fumarate; Bucindolol Hydrochloride; Bupicomide; Buthiazide: Candoxatril; Candoxatrilat; Captopril; Carvedilol; Ceronapril; Chlorothiazide Sodium; Cicletanine; Cilazapril; Clonidine; Clonidine Hydrochloride; Clopamide; Cyclopenthiazide; Cyclothiazide; Darodipine; Debrisoquin Sulfate; Delapril Hydrochloride; Diapamide; Diazoxide; Dilevalol Hydrochloride; Diltiazem Malate; Ditekiren; Doxazosin Mesylate; Ecadotril; Enalapril Maleate; Enalaprilat; Enalkiren; Endralazine Mesylate; Epithiazide; Eprosartan; Eprosartan Mesylate; Fenoldopam Mesylate; Flavodilol Maleate; Flordipine; Flosequinan; Fosinopril Sodium; Fosinoprilat; Guanabenz; Guanabenz Acetate; Guanacline Sulfate; Guanadrel Sulfate; Guancydine; Guanethidine Monosulfate; Guanethidine Sulfate; Guanfacine Hydrochloride; Guanisoquin Sulfate; Guanoclor Sulfate; Guanoctine Hydrochloride; Guanoxabenz; Guanoxan Sulfate; Guanoxyfen Sulfate; Hydralazine Hydrochloride; Hydralazine Polistirex; Hydroflumethiazide; Indacrinone; Indapamide; Indolaprif Hydrochloride; Indoramin; Indoramin Hydrochloride; Indorenate Hydrochloride; Lacidipine; Leniquinsin; Levcromakalim; Lisinopril; Lofexidine Hydrochloride; Losartan Potassium; Losulazine Hydrochloride; Mebutamate; Mecamylamine Hydrochloride; Medroxalol; Medroxalol Hydrochloride; Methalthiazide; Methyclothiazide; Methyldopa; Methyldopate Hydrochloride; Metipranolol; Metolazone; Metoprolol Fumarate; Metoprolol Succinate; Metyrosine; Minoxidil; Monatepil Maleate; Muzolimine; Nebivolol; Nitrendipine; Ofornine; Pargyline Hydrochloride; Pazoxide; Pelanserin Hydrochloride; Perindopril Erbumine; Phenoxybenzamine Hydrochloride; Pinacidil; Pivopril; Polythiazide; Prazosin Hydrochloride; Primidolol; Prizidilol Hydrochloride; Quinapril Hydrochloride; Quinaprilat; Quinazosin Hydrochloride; Quinelorane Hydrochloride; Quinpirole Hydrochloride; Quinuclium Bromide; Ramipril; Rauwolfia Serpentina; Reserpine; Saprisartan Potassium; Saralasin Acetate; Sodium Nitroprusside; Sulfinalol Hydrochloride; Tasosartan; Teludipine Hydrochloride; Temocapril Hydrochloride; Terazosin Hydrochloride; Terlakiren; Tiamenidine; Tiamenidine Hydrochloride; Ticrynafen; Tinabinol; Tiodazosin; Tipentosin Hydrochloride; Trichlormethiazide; Trimazosin Hydrochloride; Trimethaphan Camsylate; Trimoxamine Hydrochloride; Tripamide; Xipamide; Zankiren Hydrochloride; Zofenoprilat Arginine.

Anti-inflammatory: Alclofenac; Alclometasone Dipropionate; Algestone Acetonide; Alpha Amylase; Amcinafal; Amcinafide; Amfenac Sodium; Amiprilose Hydrochloride; Anakinra; Anirolac; Anitrazafen; Apazone; Balsalazide Disodium; Bendazac; Benoxaprofen; Benzydamine Hydrochloride; Bromelains; Broperamole; Budesonide; Carprofen; Cicloprofen; Cintazone; Cliprofen; Clobetasol Propionate; Clobetasone Butyrate; Clopirac; Cloticasone Propionate; Cormethasone Acetate; Cortodoxone; Deflazacort; Desonide; Desoximetasone; Dexamethasone Dipropionate; Diclofenac Potassium; Diclofenac Sodium; Diflorasone Diacetate; Diflumidone Sodium; Diflunisal; Difluprednate; Diftalone; Dimethyl Sulfoxide; Drocinonide; Endrysone; Enlimomab; Enolicam Sodium; Epirizole; Etodolac; Etofenamate; Felbinac; Fenamole; Fenbufen; Fenclofenac; Fenclorac; Fendosal; Fenpipalone; Fentiazac; Flazalone; Fluazacort; Flufenamic Acid; Flumizole; Flunisolide Acetate; Flunixin; Flunixin Meglumine; Fluocortin Butyl; Fluorometholone Acetate; Fluquazone; Flurbiprofen; Fluretofen; Fluticasone Propionate; Furaprofen; Furobufen; Halcinonide; Halobetasol Propionate; Halopredone Acetate; Ibufenac; Ibuprofen; Ibuprofen Aluminum; Ibuprofen Piconol; Ilonidap; Indomethacin; Indomethacin Sodium; Indoprofen; Indoxole; Intrazole; Isoflupredone Acetate; Isoxepac; Isoxicam; Ketoprofen; Lofemizole Hydrochloride; Lornoxicam; Loteprednol Etabonate; Meclofenamate Sodium; Meclofenamic Acid; Meclorisone Dibutyrate; Mefenamic Acid; Mesalamine; Meseclazone; Methylprednisolone Suleptanate; Momiflumate; Nabumetone; Naproxen; Naproxen Sodium; Naproxol; Nimazone; Olsalazine Sodium; Orgotein; Orpanoxin; Oxaprozin; Oxyphenbutazone; Paranyline Hydrochloride; Pentosan Polysulfate Sodium; Phenbutazone Sodium Glycerate; Pirfenidone; Piroxicam; Piroxicam Cinnamate; Piroxicam Olamine; Pirprofen; Prednazate; Prifelone; Prodolic Acid; Proquazone; Proxazole; Proxazole Citrate; Rimexolone; Romazarit; Salcolex; Salnacedin; Salsalate; Sanguinarium Chloride; Seclazone; Sermetacin; Sudoxicam; Sulindac; Suprofen; Talmetacin; Talniflumate; Talosalate; Tebufelone; Tenidap; Tenidap Sodium; Tenoxicam; Tesicam; Tesimide; Tetrydamine; Tiopinac; Tixocortol Pivalate; Tolmetin; Tolmetin Sodium; Triclonide; Triflumidate; Zidometacin; Zomepirac Sodium.

### Growth Factors

In one embodiment, an effective amount of at least one growth factor, cytokine, hormone, or extracellular matrix compound or protein useful for enhancing wound healing is administered. In one aspect, a combination of these agents is used. Growth factors useful in the practice of the disclosure include EGF, PDGF, GCSF, IL6, IL8, IL10, MCP1, MCP2, Tissue Factor, FGFb, KGF, VEGF, PLGF, MMP1, MMP9, TIMP1, TIMP2, TGFβ, and HGF. One of ordinary skill in the art will appreciate that the choice of growth factor, cytokine, hormone, or extracellular matrix protein used will vary depending on criteria such as the type of injury, disease, or disorder being treated, the age, health, sex, and weight of the subject, etc. In one aspect, the growth factors, cytokines, hormones, and extracellular matrix compounds and proteins are human.

Proteins and other biologically active compounds that can be incorporated into, or included as an additive within, a composition comprising compounds of the present disclosure include collagen (including cross-linked collagen), fibronectin, laminin, elastin (including cross-linked elastin), osteopontin, osteonectin, bone sialoproteins (Bsp), alpha-2HS-glycoproteins, bone Gla-protein (Bgp), matrix Gla-protein, bone phosphoglycoprotein, bone phosphoprotein, bone proteoglycan, protolipids, bone morphogenetic protein, cartilage induction factor, skeletal growth factor, enzymes, or combinations and biologically active fragments thereof. Adjuvants that diminish an immune response can also be used in conjunction with the composite of the subject disclosure.

Other molecules useful as compounds or substances in the present disclosure include growth hormones, leptin, leukemia inhibitory factor (LIF), tumor necrosis factor alpha and beta, endostatin, angiostatin, thrombospondin, osteogenic protein-1, bone morphogenetic proteins 2 and 7, osteonectin, somatomedin-like peptide, osteocalcin, interferon alpha, interferon alpha A, interferon beta, interferon gamma, interferon 1 alpha, and interleukins 2, 3, 4, 5, 6, 7, 8, 9, 10, 11, 12,13, 15, 16, 17 and 18. Those involving amino acids, peptides, polypeptides, and proteins may include any type of such molecules of any size and complexity as well as combinations of such molecules.

### Identification of Compounds Which Inhibit Decreased Blood Flow

Also described herein are methods for identifying compounds useful for inhibiting decreases in blood flow in a tissue associated with injury to that tissue or is associated with a disease of disorder of a tissue. Compounds which are identified using any of the methods described herein may be formulated and administered to a subject for treatment of the injuries or diseases disclosed herein. For example, test compounds may be added at varying doses and frequencies to determine the effective amount of the compound which should be used and effective intervals in which it should be administered. In another aspect, a derivative or modification of the test compound may be used. One of ordinary skill in the art will recognize that these methods will be useful for other diseases, disorders, and conditions as well.

In one example, the exposed small intestine mesenteric vessel model is used to identify compounds which can inhibit decreased blood flow associated with an injury.

### Pharmaceutical Compositions and Delivery Form

The formulations described herein may be prepared in a variety of forms known in the art, such as liquids, aerosols, or gels. Topical administration of the present formulation can be performed by, for example, hand, mechanically (e.g., extrusion and spray delivery) or as a component of a dressing (*e.g*., gauze or other wound covering). The administration of the formulation directly by hand to a tissue or biomaterial surface is preformed so as to achieve a therapeutic coating, which may be uniform, alone or in combination with an overlying dressing.

In one example, the administration of the formulation mechanically is performed by using a device that physically pushes the composition onto a tissue or biomaterial surface so as to achieve a therapeutic coating, which may be uniform, alone or in combination with an overlying dressing.

In another example, the formulation can be sprayed onto a tissue or biomaterial surface so as to achieve a therapeutic coating, which may be uniform, alone or in combination with an overlying dressing. When part of a dressing, the formulation is applied so as to achieve a therapeutic coating of the surface, which may be uniform.

Formulations suitable for topical administration include liquid or semi-liquid preparations such as liniments, lotions, oil-in-water or water-in-oil emulsions such as creams, ointments or pastes, and solutions or suspensions. Topically-administrable formulations may, for example, comprise from about 1% to about 70% (w/w) active ingredient, although the concentration of the active ingredient may be as high as the solubility limit of the active ingredient in the solvent. Formulations for topical administration may further comprise one or more of the additional ingredients described herein.

In one example, a pharmaceutical cream is provided wherein a poloxamer base, in the form of powder, is mixed with water, and caused to become hydrated, by subjecting the combination of poloxamer base and water, to freezing temperatures, before a pharmaceutical agent such as an additional therapeutic agent is added.

Those of ordinary skill in the art will be able to identify readily those pharmaceutical agents that have utility with the present disclosure. Those of ordinary skill in the art will also recognize numerous other compounds that fall within the categories and that are useful according to the present disclsoure for treating injuries where reduced blood flow occurs.

The disclosure encompasses the preparation and use of pharmaceutical compositions comprising a compound for use in the treatment of various skin related injuries, trauma, diseases, disorders, or conditions described herein, including bums, wounds, surgical incisions. The present disclosure also encompasses other injuries, trauma, associated diseases and disorders other than those of the skin, including gum diseases and disorders. Such a pharmaceutical composition may consist of the active ingredient alone, in a form suitable for administration to a subject, or the pharmaceutical composition may comprise at least one active ingredient and one or more pharmaceutically acceptable carriers, one or more additional ingredients, or some combination of these. The active ingredient may be present in the pharmaceutical composition in the form of a physiologically acceptable ester or salt, such as in combination with a physiologically acceptable cation or anion, as is well known in the art.

An obstacle for topical administration of pharmaceuticals to the skin is the stratum corneum layer of the epidermis. The stratum corneum is a highly resistant layer comprised of protein, cholesterol, sphingolipids, free fatty acids and various other lipids, and includes cornified and living cells. One of the factors that limits the penetration rate (flux) of a compound through the stratum corneum is the amount of the active substance which can be loaded or applied onto the skin surface. The greater the amount of active substance which is applied per unit of area of the skin, the greater the concentration gradient between the skin surface and the lower layers of the skin, and in turn the greater the diffusion force of the active substance through the skin. Therefore, a formulation containing a greater concentration of the active substance is more likely to result in penetration of the active substance through the skin, and more of it, and at a more consistent rate, than a formulation having a lesser concentration, all other things being equal.

The formulations of the pharmaceutical compositions described herein may be prepared by any method known or hereafter developed in the art of pharmacology. In general, such preparatory methods include the step of bringing the active ingredient into association with a carrier or one or more other accessory ingredients, and then, if necessary or desirable, shaping or packaging the product into a desired single- or multi-dose unit.

The compounds of the disclosure may be administered to, for example, a cell, a tissue, or a subject by any of several methods described herein and by others which are known to those of skill in the art.

The relative amounts of the active ingredient, the pharmaceutically acceptable carrier, and any additional ingredients in a pharmaceutical composition of the disclosure will vary, depending upon the identity, sex, age, size, and condition of the subject treated and further depending upon the route by which the composition is to be administered.

In addition to the active ingredient, a pharmaceutical composition of the invention may further comprise one or more additional pharmaceutically active or therapeutic agents. Particularly contemplated additional agents include anti-emetics and scavengers such as cyanide and cyanate scavengers.

Controlled- or sustained-release formulations of a pharmaceutical composition of the disclosure may be made using conventional technology. Formulations suitable for topical administration include liquid or semi-liquid preparations such as liniments, lotions, oil-in-water or water-in-oil emulsions such as creams, ointments or pastes, and solutions or suspensions. Topically-administrable formulations may, for example, comprise from about 1% to about 10% (w/w) active ingredient, although the concentration of the active ingredient may be as high as the solubility limit of the active ingredient in the solvent. Formulations for topical administration may further comprise one or more of the additional ingredients described herein.

Additionally, formulations for topical administration may include liquids, ointments, lotions, creams, gels (e.g., poloxamer gel), drops, suppositories, sprays, aerosols, and powders. Conventional pharmaceutical carriers, aqueous, powder or oily bases and thickeners may be necessary or desirable. The disclosed compositions can be administered, for example, in a microfiber, polymer (e.g., collagen), nanosphere, aerosol, lotion, cream, fabric, plastic, tissue engineered scaffold, matrix material, tablet, implanted container, powder, oil, resin, wound dressing, bead, microbead, slow release bead, capsule, injectables, intravenous drips, pump device, silicone implants, or any bio-engineered materials.

Enhancers of permeation may be used. These materials increase the rate of penetration of drugs across the skin. Typical enhancers in the art include ethanol, glycerol monolaurate, PGML (polyethylene glycol monolaurate) and dimethylsulfoxide. Other enhancers include oleic acid, oleyl alcohol, ethoxydiglycol, laurocapram, alkanecarboxylic acids, dimethylsulfoxide, polar lipids, or N-methyl-2-pyrrolidone.

One acceptable vehicle for topical delivery of some of the compositions of the disclosure may contain liposomes. The composition of the liposomes and their use are known in the art (for example, see Constanza, U.S. Pat. No. 6,323,219).

The source of active compound to be formulated will generally depend upon the particular form of the compound. Small organic molecules and peptidyl or oligo fragments can be chemically synthesized and provided in a pure form suitable for pharmaceutical/cosmetic usage. Products of natural extracts can be purified according to techniques known in the art. Recombinant sources of compounds are also available to those of ordinary skill in the art.

The topically active pharmaceutical composition may be optionally combined with other ingredients such as moisturizers, cosmetic adjuvants, antioxidants, chelating agents, bleaching agents, tyrosinase inhibitors, and other known depigmentation agents, surfactants, foaming agents, conditioners, humectants, wetting agents, emulsifying agents, fragrances, viscosifiers, buffering agents, preservatives and sunscreens. In another example, a permeation or penetration enhancer is included in the composition and is effective in improving the percutaneous penetration of the active ingredient into and through the stratum corneum with respect to a composition lacking the permeation enhancer. Various permeation enhancers, including oleic acid, oleyl alcohol, ethoxydiglycol, laurocapram, alkanecarboxylic acids, dimethylsulfoxide, polar lipids, or N-methyl-2-pyrrolidone, are known to those of skill in the art. The composition may further comprise a hydrotropic agent, which functions to increase disorder in the structure of the stratum corneum, and thus allows increased transport across the stratum corneum. Various hydrotropic agents such as isopropyl alcohol, propylene glycol, or sodium xylene sulfonate, are known to those of skill in the art. The compositions may also contain active amounts of retinoids (i.e., compounds that bind to any members of the family of retinoid receptors), including, for example, tretinoin, retinol, esters of tretinoin and/or retino 1. Although the descriptions of pharmaceutical compositions provided herein are principally directed to pharmaceutical compositions which are suitable for ethical administration to humans, it will be understood by the skilled artisan that such compositions are generally suitable for administration to animals of all sorts.

The present disclosure encompasses biologically active analogs, homo logs, derivatives, and modifications of the compounds described herein. Methods for the preparation of such compounds are known in the art.

Modification of pharmaceutical compositions suitable for administration to humans in order to render the compositions suitable for administration to various animals is well understood, and the ordinarily skilled veterinary pharmacologist can design and perform such modification with merely ordinary, if any, experimentation. Subjects to which administration of the pharmaceutical compositions of the invention is contemplated include humans and other primates, mammals including commercially relevant mammals such as cattle, pigs, horses, sheep, cats, and dogs.

Liquid derivatives and natural extracts made directly from biological sources may be employed in the compositions of this disclosure in a concentration (w/w) from about 1 to about 99%. Fractions of natural extracts and protease inhibitors may have a different preferred rage, from about 0.01 % to about 20% and, more preferably, from about 1% to about 10% of the composition. Of course, mixtures of the active agents of this disclosure may be combined and used together in the same formulation, or in serial applications of different formulations.

The composition may comprise a preservative from about 0.005% to 2.0% by total weight of the composition. The preservative is used to prevent spoilage in the case of an aqueous gel because of repeated patient use when it is exposed to contaminants in the environment from, for example, exposure to air or the patient's skin, including contact with the fingers used for applying a composition of the invention such as a therapeutic gel or cream. Examples of preservatives useful in accordance with the present disclosure include those selected from the group consisting of benzyl alcohol, sorbic acid, parabens, imidurea, and combinations thereof. A particularly preferred preservative is a combination of about 0.5% to 2.0% benzyl alcohol and 0.05% to 0.5% sorbic acid.

The composition may include an antioxidant and a chelating agent which inhibit the degradation of the compound for use in the invention in the aqueous gel formulation. Preferred antioxidants for some compounds are BHT, BHA, alphatocopherol, and ascorbic acid in the preferred range of about 0.01% to 0.3% and more preferably BHT in the range of 0.03% to 0.1% by weight by total weight of the composition. Preferably, the chelating agent is present in an amount of from 0.01% to 0.5% by weight by total weight of the composition. Particularly preferred chelating agents include edetate salts (e.g. disodium edetate) and citric acid in the weight range of about 0.01% to 0.20% and more preferably in the range of 0.02% to 0.10% by weight by total weight of the composition. The chelating agent is useful for chelating metal ions in the composition which may be detrimental to the shelf life of the formulation. While BHT and disodium edetate are preferred antioxidant and chelating agent respectively for some compounds, other suitable and equivalent antioxidants and chelating agents may be substituted therefor as would be known to those skilled in the art.

As used herein, "additional ingredients" include one or more of the following: excipients; surface active agents; dispersing agents; inert diluents; granulating and disintegrating agents; binding agents; lubricating agents; sweetening agents; flavoring agents; coloring agents; preservatives; physiologically degradable compositions such as gelatin; aqueous vehicles and solvents; oily vehicles and solvents; suspending agents; dispersing or wetting agents; emulsifying agents, demulcents; buffers; salts; thickening agents; fillers; emulsifying agents; antioxidants; antibiotics; antifungal agents; stabilizing agents; and pharmaceutically acceptable polymeric or hydrophobic materials. Other "additional ingredients" which may be included in the pharmaceutical compositions are known in the art and described, for example in Genaro, ed. (1985, Remington's Pharmaceutical Sciences, Mack Publishing Co., Easton, Pa.).

Other components such as preservatives, antioxidants, surfactants, absorption enhancers, viscosity enhancers or film forming polymers, bulking agents, diluents, coloring agents, flavoring agents, pH modifiers, sweeteners or taste-masking agents may also be incorporated into the composition. Suitable coloring agents include red, black, and yellow iron oxides and FD&C dyes such as FD&C Blue No. 2 and FD&C Red No. 40. Suitable flavoring agents include mint, raspberry, licorice, orange, lemon, grapefruit, caramel, vanilla, cherry grape flavors and combinations thereof. Suitable pH modifiers include citric acid, tartaric acid, phosphoric acid, hydrochloric acid, maleic acid and sodium hydroxide. Suitable sweeteners include aspartame, acesulfame K and thaumatin. Suitable taste-masking agents include sodium bicarbonate, ion-exchange resins, cyclodextrin inclusion compounds and adsorbates. Absorption enhancers for use in accordance with the present dislosure include, for example, polysorbates, sorbitan esters, poloxamer block copolymers, PEG-35 castor oil, PEG-40 hydrogenated castor oil, caprylocaproyl macrogol-8 glycerides, PEG-8 caprylic/capric glycerides, sodium lauryl sulfate, dioctyl sulfosuccinate, polyethylene lauryl ether, ethoxydiglycol, propylene glycol mono-dicaprylate, glycerol monocaprylate, glyceryl fatty acids, oleic acid, linoleic acid, glyceryl caprylate/caprate, glyceryl monooleate, glyceryl monolaurate, caprylic/capric triglycerides, ethoxylated nonylphenols, PEG-(8-50) stearates, olive oil PEG-6 esters, triolein PEG-6 esters, lecithin, d-alpha tocopheryl polyethylene glycol 1000 succinate, polycarbonate, sodium glycocholate, sodium taurocholate, cyclodextrins, citric acid, sodium citrate, triacetin and combinations thereof. A preferred absorption enhancer is triacetin. A preferred absorption enhancer wherein an absorption enhancer is included in the formulation, the absorption enhancer is included in an amount of from about 0.001% to about 10% by weight of the formulation, preferably in an amount of about 0.01% to about 5% by weight of the formulation.

The formulations of the pharmaceutical compositions described herein may be prepared by any method known or hereafter developed in the art of pharmacology. In general, such preparatory methods include the step of bringing the active ingredient into association with a carrier or one or more other accessory ingredients, and then, if necessary or desirable, shaping or packaging the product into a desired single- or multi-dose unit.

Although the descriptions of pharmaceutical compositions provided herein are principally directed to pharmaceutical compositions which are suitable for ethical administration to humans, it will be understood by the skilled artisan that such compositions are generally suitable for administration to animals of all sorts. Modification of pharmaceutical compositions suitable for administration to humans in order to render the compositions suitable for administration to various animals is well understood, and the ordinarily skilled veterinary pharmacologist can design and perform such modification with merely ordinary, if any, experimentation. Subjects to which administration of the pharmaceutical compositions of the disclosure is contemplated include humans and other primates, mammals including commercially relevant mammals such as cattle, pigs, horses, sheep, cats, and dogs, and birds including commercially relevant birds such as chickens, ducks, geese, and turkeys.

The pharmaceutical compositions of the disclosure can be administered in any suitable formulation, by any suitable means, and by any suitable route of administration. Formulations suitable for topical administration liquid or semi-liquid preparations such as liniments, lotions, oil in water or water in oil emulsions such as creams, ointments or pastes, and solutions or suspensions. Topically-administrable formulations may, for example, comprise from about 1% to about 10% (w/w) active ingredient, although the concentration of the active ingredient may be as high as the solubility limit of the active ingredient in the solvent. Formulations for topical administration may further comprise one or more of the additional ingredients described herein.

Topical administration of compositions of the invention may include transdermal application. Transdermal application can be performed either passively or using iontophoresis or electroporation.

Compositions of the invention may be applied using transdermal patches. Transdermal patches are adhesive backed patches laced with an effective amount of compounds of the invention. The pressure-sensitive adhesive of the matrix will normally be a solution of polyacrylate, a silicone, or polyisobutylene (PIB). Such adhesives are well known in the transdermal art. See, for instance, the Handbook of Pressure Sensitive Adhesive Technology, 2nd Edition (1989) Van Nostrand, Reinhold.

Pressure sensitive solution polyacrylate adhesives for transdermal patches are made by copolymerizing one or more acrylate monomers ("acrylate" is intended to include both acrylates and methacrylates), one or more modifying monomers, and one or more functional group-containing monomers in an organic solvent. The acrylate monomers used to make these polymers are normally alkyl acrylates of 4-17 carbon atoms, with 2-ethylhexyl acrylate, butyl acrylate, and isooctyl acrylate being preferred. Modifying monomers are typically included to alter the Tg of the polymer. Such monomers as vinyl acetate, ethyl acrylate and methacrylate, and methyl methacrylate are useful for this purpose. The functional group-containing monomer provides sites for crosslinking. The functional groups of these monomers are preferably carboxyl, hydroxy or combinations thereof. Examples of monomers that provide such groups are acrylic acid, methacrylic acid and hydroxy-containing monomers such as hydroxyethyl acrylate. The polyacrylate adhesives are preferably crosslinked using a crosslinking agent to improve their physical properties, (e.g., creep and shear resistance). The crosslinking density should be low since high degrees of crosslinking may affect the adhesive properties of the copolymer adversely. Examples of crosslinking agents are disclosed in U.S. Pat. No. 5,393,529. Solution polyacrylate pressure sensitive adhesives are commercially available under tradenames such as GELVA™ and DURO-TAK™ from 3M.

Polyisobutylene adhesives are mixtures of high molecular weight (HMW) PIB and low molecular weight (LMW) PIB. Such mixtures are described in the art, e.g., PCT/US91/02516. The molecular weight of the HMW PIB will usually be in the range of about 700,000 to 2,000,000 Da, whereas that of the LMW PIB will typically range between 35,000 to 60,000. The molecular weights referred to herein are weight average molecular weight. The weight ratio of HMW PIB to LMW PIB in the adhesive will normally range between 1:1 to 1:10. The PIB adhesive will also normally include a tackifier such as polybutene oil and high Tg, low molecular weight aliphatic resins such as the ESCOREZ™ resins available from Exxon Chemical. Polyisobutylene polymers are available commercially under the tradename VISTANEX™ from Exxon Chemical.
The silicone adhesives that may be used in forming the matrix are typically high molecular weight polydimethyl siloxanes or polydimethyldiphenyl siloxanes. Formulations of silicone adhesives that are useful in transdermal patches are described in U.S. Pat. Nos. 5,232,702, 4,906,169, and 4,951,622.

Dosage forms for topical or transdermal administration of a compound of this disclosure include liquids, ointments, pastes, creams, lotions, gels, powders, solutions, sprays, aerosols, inhalants or patches. The active component is admixed under sterile conditions with a pharmaceutically acceptable carrier and any needed preservatives or buffers as may be required. Ophthalmic formulation, ear drops, eye ointments, powders, and solutions are also contemplated as being within the scope of this disclosure. Transdermal patches have the added advantage of providing controlled delivery of a compound to the body. Such dosage forms can be made by dissolving or dispensing the compound(s) in the proper medium. Absorption enhancers can also be used to increase the flux of the compound across the skin. The rate can be controlled by either providing a rate controlling membrane or by dispersing the compound in a polymer matrix or gel.

The ointments, pastes, creams, and gels may contain, in addition to an active compound of this disclosure excipients such as animal and vegetable fats, oils, waxes, paraffins, starch, tragacanth, cellulose derivatives, polyethylene glycols, silicones, bentonites, silicic acid, talc and zinc oxide, or mixtures thereof.

Topical administration may also be performed using iontophoresis devices. Such delivery systems eliminate needles entirely, and rely upon chemical mediators or external driving forces such as iontophoretic currents or thermal poration or sonophoresis to breach the stratum corneum, the outermost layer of the skin, and deliver substances through the surface of the skin. The process of iontophoresis has found commercial use in the delivery of ionically charged therapeutic agent molecules such as pilocarpine, lidocaine, and dexamethasone. In this delivery method, ions bearing a positive charge are driven across the skin at the site of an electrolytic electrical system anode while ions bearing a negative charge are driven across the skin at the site of an electrolytic system cathode.

The present disclosure provides a system for the direct application of compounds of the disclosure, including additional therapeutic agents such as anesthetic agents, by iontophoresis for the treatment of decreased blood flow and concurrent pain associated with injuries, diseases, and disorders. While many compounds may be useful with the invention, as will be discussed below, it is particularly useful for the delivery of anesthetic agents such as lidocaine, bupivicaine, ropivicaine, and mepivicaine to damaged skin.

Alos described are methods to provide a patch device with a donor or delivery chamber that is designed to be applied directly over an injury, incision, or wound site and utilizes an electric field to stimulate delivery of the active compound or additional therapeutic agents(s). The patch is sterilized so that risk of infection is minimal. Additionally, the system delivers medication in a constant manner over an extended period of time. Generally, such time periods are at least 30 minutes and may extend to as many as 96 hours.

A pharmaceutical composition of the disclosure may be prepared, packaged, or sold in a formulation suitable for pulmonary administration via the buccal cavity. Such a formulation may comprise dry particles which comprise the active ingredient and which have a diameter in the range from about 0.5 to about 7 nanometers, and preferably from about 1 to about 6 nanometers. Such compositions are conveniently in the form of dry powders for administration using a device comprising a dry powder reservoir to which a stream of propellant may be directed to disperse the powder or using a self-propelling solvent/powder-dispensing container such as a device comprising the active ingredient dissolved or suspended in a low-boiling propellant in a sealed container. Preferably, such powders comprise particles wherein at least 98% of the particles by weight have a diameter greater than 0.5 nanometers and at least 95% of the particles by number have a diameter less than 7 nanometers. More preferably, at least 95% of the particles by weight have a diameter greater than 1 nanometer and at least 90% of the particles by number have a diameter less than 6 nanometers. Dry powder compositions preferably include a solid fine powder diluent such as sugar and are conveniently provided in a unit dose form.

Low boiling propellants generally include liquid propellants having a boiling point of below 65°F at atmospheric pressure. Generally, the propellant may constitute about 50% to about 99.9% (w/w) of the composition, and the active ingredient may constitute about 0.1% to about 20% (w/w) of the composition. The propellant may further comprise additional ingredients such as a liquid non-ionic or solid anionic surfactant or a solid diluent (preferably having a particle size of the same order as particles comprising the active ingredient).

Pharmaceutical compositions of the disclosure formulated for pulmonary delivery may also provide the active ingredient in the form of droplets of a solution or suspension. Such formulations may be prepared, packaged, or sold as aqueous or dilute alcoholic solutions or suspensions, optionally sterile, comprising the active ingredient, and may conveniently be administered using any nebulization or atomization device. Such formulations may further comprise one or more additional ingredients including a flavoring agent such as saccharin sodium, a volatile oil, a buffering agent, a surface active agent, or a preservative such as methylhydroxybenzoate. The droplets provided by this route of administration preferably have an average diameter in the range from about 0.1 to about 200 nanometers.

The formulations described herein as being useful for pulmonary delivery are also useful for intranasal delivery of a pharmaceutical composition of the disclosure.

Another formulation suitable for intranasal administration is a coarse powder comprising the active ingredient and having an average particle from about 0.2 to about 500 micrometers. Such a formulation is administered in the manner in which snuff is taken, i.e., by rapid inhalation through the nasal passage from a container of the powder held close to the nares.

Formulations suitable for nasal administration may, for example, comprise from about as little as about 0.1% (w/w) and as much as about 100% (w/w) of the active ingredient, and may further comprise one or more of the additional ingredients described herein.

A pharmaceutical composition of the disclosure may be prepared, packaged, or sold in a formulation suitable for buccal administration. Such formulations may, for example, be in the form of tablets or lozenges made using conventional methods, and may, for example, comprise about 0.1% to about 20% (w/w) active ingredient, the balance comprising an orally dissolvable or degradable composition and, optionally, one or more of the additional ingredients described herein. Alternately, formulations suitable for buccal administration may comprise a powder or an aerosolized or atomized solution or suspension comprising the active ingredient. Such powdered, aerosolized, or atomized formulations, when dispersed, preferably have an average particle or droplet size in the range from about 0.1 to about 200 nanometers, and may further comprise one or more of the additional ingredients described herein. Additionally, the formulation taken orally can be prepared as a pharmaceutical composition, including a paste, a gel, a toothpaste, a mouthwash, a solution, an oral rinse, a suspension, an ointment, a cream, and a coating.

A pharmaceutical composition of the disclosure may be prepared, packaged, or sold in a formulation suitable for ophthalmic administration. Such formulations may, for example, be in the form of eye drops including, for example, a 0.1% to 1.0% (w/w) solution or suspension of the active ingredient in an aqueous or oily liquid carrier. Such drops may further comprise buffering agents, salts, or one or more other of the additional ingredients described herein. Other opthalmically-administrable formulations which are useful include those which comprise the active ingredient in microcrystalline form or in a liposomal preparation.

A pharmaceutical composition of the disclosure may be prepared, packaged, or sold in a formulation suitable for intramucosal administration. Such intramucosal administration of compounds allows passage or absorption of the compounds across mucosa. Such type of administration is useful for absorption orally (gingival, sublingual, buccal, etc.), rectally, vaginally, pulmonary, nasally, etc.

In some examples, sublingual administration has an advantage for active ingredients, as well as additional therapeutic agents, which in some cases, when given orally, are subject to a substantial first pass metabolism and enzymatic degradation through the liver, resulting in rapid metabolization and a loss of therapeutic activity related to the activity of the liver enzymes that convert the molecule into inactive metabolites, or the activity of which is decreased because of this bioconversion.

In some cases, a sublingual route of administration is capable of producing a rapid onset of action due to the considerable permeability and vascularization of the buccal mucosa. Moreover, sublingual administration can also allow the administration of active ingredients which are not normally absorbed at the level of the stomach mucosa or digestive mucosa after oral administration, or alternatively which are partially or completely degraded in acidic medium after ingestion of, for example, a tablet.

The compounds of the disclosure can be prepared in a formulation or pharmaceutical composition appropriate for administration that allows or enhances absorption across mucosa. Mucosal absorption enhancers a bile salt, fatty acid, surfactant, or alcohol. Permeation enhancers can be sodium cholate, sodium dodecyl sulphate, sodium deoxycholate, taurodeoxycholate, sodium glycocholate, dimethylsulfoxide, or ethanol. A compound of the disclosure can be formulated with a mucosal penetration enhancer to facilitate delivery of the compound. The formulation can also be prepared with pH optimized for solubility, drug stability, and absorption through mucosa such as nasal mucosa, oral mucosa, vaginal mucosa, respiratory, and intestinal mucosa.

To further enhance mucosal delivery of pharmaceutical agents, formulations comprising the active agent may also contain a hydrophilic low molecular weight compound as a base or excipient. Such hydrophilic low molecular weight compounds provide a passage medium through which a water-soluble active agent, such as a physiologically active peptide or protein, may diffuse through the base to the body surface where the active agent is absorbed. The hydrophilic low molecular weight compound optionally absorbs moisture from the mucosa or the administration atmosphere and dissolves the water-soluble active peptide. The molecular weight of the hydrophilic low molecular weight compound is generally not more than 10000 and preferably not more than 3000. Exemplary hydrophilic low molecular weight compounds include polyol compounds, such as oligo-, di- and monosaccharides such as sucrose, mannitol, lactose, L-arabinose, D-erythrose, D-ribose, D-xylose, D-mannose, D-galactose, lactulose, cellobiose, gentibiose, glycerin, and polyethylene glycol. Other examples of hydrophilic low molecular weight compounds useful as carriers include N-methylpyrrolidone, and alcohols (e.g., oligovinyl alcohol, ethanol, ethylene glycol, propylene glycol, etc.). These hydrophilic low molecular weight compounds can be used alone or in combination with one another or with other active or inactive components of the intranasal formulation.

When a controlled-release pharmaceutical preparation of the present disclosure further contains a hydrophilic base, many options are available for inclusion. Hydrophilic polymers such as a polyethylene glycol and polyvinyl pyrrolidone, sugar alcohols such as D-sorbitol and xylitol, saccharides such as sucrose, maltose, lactulose, D-fructose, dextran, and glucose, surfactants such as polyoxyethylene-hydrogenated castor oil, polyoxyethylene polyoxypropylene glycol, and polyoxyethylene sorbitan higher fatty acid esters, salts such as sodium chloride and magnesium chloride, organic acids such as citric acid and tartaric acid, amino acids such as glycine, beta-alanine, and lysine hydrochloride, and amino saccharides such as meglumine are given as examples of the hydrophilic base. Polyethylene glycol, sucrose, and polyvinyl pyrrolidone are preferred and polyethylene glycol are further preferred. One or a combination of two or more hydrophilic bases can be used in the present disclosure. The present disclosure contemplates pulmonary, nasal, or oral administration through an inhaler. In one example, delivery from an inhaler can be a metered dose.

An inhaler is a device for patient self-administration of at least one compound of the disclosure comprising a spray inhaler (e.g., a nasal, oral, or pulmonary spray inhaler) containing an aerosol spray formulation of at least one compound of the disclosure and a pharmaceutically acceptable dispersant. In one example, the device is metered to disperse an amount of the aerosol formulation by forming a spray that contains a dose of at least one compound of the disclosure effective to treat a disease or disorder. The dispersant may be a surfactant, such as, polyoxyethylene fatty acid esters, polyoxyethylene fatty acid alcohols, and polyoxyethylene sorbitan fatty acid esters. Phospholipid-based surfactants also may be used.

In other examples, the aerosol formulation is provided as a dry powder aerosol formulation in which a compound of the disclosure is present as a finely divided powder. The dry powder formulation can further comprise a bulking agent, such as lactose, sorbitol, sucrose, and mannitol.

In another specific example, the aerosol formulation is a liquid aerosol formulation further comprising a pharmaceutically acceptable diluent, such as sterile water, saline, buffered saline and dextrose solution.

In further examples, the aerosol formulation further comprises at least one additional compound of the disclosure in a concentration such that the metered amount of the aerosol formulation dispersed by the device contains a dose of the additional compound in a metered amount that is effective to ameliorate the symptoms of disease or disorder disclosed herein when used in combination with at least a first or second compound of the present disclosure.

Compounds of the disclosure will be prepared in a formulation or pharmaceutical composition appropriate for nasal administration. The compounds of the disclosure can be formulated with a mucosal penetration enhancer to facilitate delivery of the drug. The formulation can also be prepared with pH optimized for solubility, drug stability, absorption through nasal mucosa, and other considerations.

Capsules, blisters, and cartridges for use in an inhaler or insufflator may be formulated to contain a powder mix of the pharmaceutical compositions provided herein; a suitable powder base, such as lactose or starch; and a performance modifier, such as l-leucine, mannitol, or magnesium stearate. The lactose may be anhydrous or in the form of the monohydrate. Other suitable excipients include dextran, glucose, maltose, sorbitol, xylitol, fructose, sucrose, and trehalose. The pharmaceutical compositions provided herein for inhaled/intranasal administration may further comprise a suitable flavor, such as menthol and levomenthol, or sweeteners, such as saccharin or saccharin sodium.

For administration by inhalation, the compounds of the disclosure are conveniently delivered in the form of an aerosol spray presentation from pressurized packs or a nebulizer, with the use of a suitable propellant, e.g., dichlorodifluoromethane, trichlorofluoromethane, dichlorotetrafluoroethane, carbon dioxide, or other suitable gas. In the case of a pressurized aerosol, the dosage unit may be determined by providing a valve to deliver a metered amount. Capsules and cartridges of e.g., gelatin for use in an inhaler or insufflator may be formulated containing a powder mix of the drugs and a suitable powder base such as lactose or starch.

As used herein, "additional ingredients" include one or more of the following: excipients; surface active agents; dispersing agents; inert diluents; granulating and disintegrating agents; binding agents; lubricating agents; sweetening agents; flavoring agents; coloring agents; preservatives; physiologically degradable compositions such as gelatin; aqueous vehicles and solvents; oily vehicles and solvents; suspending agents; dispersing or wetting agents; emulsifying agents, demulcents; buffers; salts; thickening agents; fillers; emulsifying agents; antioxidants; antibiotics; antifungal agents; stabilizing agents; and pharmaceutically acceptable polymeric or hydrophobic materials. Other "additional ingredients" which may be included in the pharmaceutical compositions of the disclosure are known in the art and described, for example in Genaro, ed., 1985, Remington's Pharmaceutical Sciences, Mack Publishing Co., Easton, PA.

Typically, dosages of the compounds of the disclosure which may be administered to an animal, preferably a human, range in amount from about 1.0 µg to about 100 g per kilogram of body weight of the animal. The precise dosage administered will vary depending upon any number of factors including the type of animal and type of disease state being treated, the age of the animal and the route of administration. Preferably, the dosage of the compound will vary from about 1 mg to about 10 g per kilogram of body weight of the animal. More preferably, the dosage will vary from about 10 mg to about 1 g per kilogram of body weight of the animal.

The compounds may be administered to a subject as frequently as several times daily, or it may be administered less frequently, such as once a day, once a week, once every two weeks, once a month, or even less frequently, such as once every several months or even once a year or less. The frequency of the dose will be readily apparent to the skilled artisan and will depend upon any number of factors, such as the type and severity of the disease being treated, the type and age of the animal.

### Use of cells

The present disclosure in which the treatment comprises cells include cells that can be cultured in vitro, derived from a natural source, genetically engineered, or produced by any other means.

Some examples use cells that have been genetically engineered. The engineering involves programming the cell to express one or more genes, repressing the expression of one or more genes, or both. One example of genetically engineered cells useful herein is a genetically engineered cell that makes and secretes one or more desired molecules. Cells may produce substances to aid in the following non-inclusive list of purposes: inhibit or stimulate inflammation; facilitate healing; resist immunorejection; provide hormone replacement; replace neurotransmitters; inhibit or destroy cancer cells; promote cell growth; inhibit or stimulate formation of blood vessels; augment tissue; and to supplement or replace neurons, skin, synovial fluid, tendons, cartilage including articular cartilage), ligaments, bone, muscle, organs, dura, blood vessels, bone marrow, and extracellular matrix. Various growth factors, cytokines, or other molecules may also be administered to regulate the cell and/or aid in the function of interest of that cell.

The cells of the present disclosure may be administered to a subject alone or in admixture with a composition useful in the repair of wounds and other defects. Such compositions include bone morphogenetic proteins, hydroxyapatite/tricalcium phosphate particles (HA/TCP), gelatin, poly-L-lysine, and collagen.

Cells of the present disclosure can be used in conjunction with a product such as Dermagraft.® Dermagraft® is indicated for use in the treatment of full-thickness diabetic foot ulcers greater than six weeks duration, which extend through the dermis, but without tendon, muscle, joint capsule, or bone exposure. Dermagraft® is a cryopreserved human fibroblast-derived dermal substitute; it is composed of fibroblasts, extracellular matrix, and a bioabsorbable scaffold. Dermagraft® is manufactured from human fibroblast cells derived from newborn foreskin tissue. During the manufacturing process, the human fibroblasts are seeded onto a bioabsorbable polyglactin mesh scaffold. The fibroblasts proliferate to fill the interstices of this scaffold and secrete human dermal collagen, matrix proteins, growth factors, and cytokines to create a three-dimensional human dermal substitute containing metabolically active living cells. Dermagraft® does not contain macrophages, lymphocytes, blood vessels, or hair follicles.

The present disclosure provides a method of promoting the closure of a wound within a subject using cells and compositions as described herein. In accordance with the method, the cells which have been selected or have been modified to secrete a hormone, growth factor, or other agent are transferred to the vicinity of a wound under conditions sufficient for the cell to produce the hormone, growth factor or other agent. The presence of the hormone, growth factor, or other agent in the vicinity of the wound promotes closure of the wound. In one example, proliferation of the administered cells promotes healing of the wound. In one example, differentiation of the administered cells promotes healing of the wound. The method promotes closure of both external (e.g., surface) and internal wounds. Wounds to which the present disclosure method is useful in promoting closure include abrasions, avulsions, blowing wounds, burn wounds, contusions, gunshot wounds, incised wounds, open wounds, penetrating wounds, perforating wounds, puncture wounds, seton wounds, stab wounds, surgical wounds, subcutaneous wounds, diabetic lesions, or tangential wounds. The method need not achieve complete healing or closure of the wound; it is sufficient for the method to promote any degree of wound closure. In this respect, the method can be employed alone or as an adjunct to other methods for healing wounded tissue.

The present disclosure provides a method of treating a disorder amenable to cell therapy comprising administering to the affected subject a therapeutically effective amount of the cells described herein.

In one example, the cells are obtained and cultured in order to derive and store the cells for therapeutic uses using cell therapy should the subject require, for example, disease therapy, tissue repair, transplantation, treatment of a cellular debilitation, or treatment of cellular dysfunctions in the future.

In another example, cells derived from a subject are directly differentiated in vitro or in vivo to generate differentiating or differentiated cells without generating a cell line.

Such cell therapy methods encompass the use of the cells described herein in combination with growth factors or chemokines such as those inducting proliferation, lineage-commitment, or genes or proteins of interest. Treatment methods may include providing stem or appropriate precursor cells directly for transplantation where the tissue is regenerated in vivo or recreating the desired tissue in vitro and then providing the tissue to the affected subject.

The composites and/or cells of the present disclosure can be used as a vehicle for the in situ delivery of biologically active agents. The biologically active agents incorporated into, or included as an additive within, the composite of the subject invention can include, without limitation, medicaments, growth factors, vitamins, mineral supplements, substances used for the treatment, prevention, diagnosis, cure or mitigation of disease or illness, substances which affect the structure or function of the body, or drugs. The biologically active agents can be used, for example, to facilitate implantation of the composite or cell suspension into a subject to promote subsequent integration and healing processes. The active agents include antifungal agents, antibacterial agents, anti-viral agents, anti-parasitic agents, growth factors, angiogenic factors, anesthetics, mucopolysaccharides, metals, cells, and other wound healing agents. Because the processing conditions can be relatively benign (physiological temperature and pH), live cells can be incorporated into the composite during its formation, or subsequently allowed to infiltrate the composite through tissue engineering techniques.

In one example, a composition comprising the cells of the present disclosure is administered locally by injection. Compositions comprising the cells can be further combined with known drugs, and in one embodiment, the drugs are bound to the cells. These compositions can be prepared in the form of an implantable device that can be molded to a desired shape. In one example, a graft construct is prepared comprising a biocompatible matrix and one or more cells of the present disclosure, wherein the matrix is formed in a shape to fill a gap or space created by the removal of a tumor, injured, or diseased tissue.

The cells can be seeded onto the desired site within the tissue to establish a population. Cells can be transferred to sites in vivo using devices such as catheters, trocars, cannulae, stents (which can be seeded with the cells), etc.

The cells can be employed alone or within biologically-compatible compositions to generate differentiated tissues and structures, both in vivo and in vitro, or to stimulate a process of interest in a tissue. Additionally, the cells can be expanded and cultured to produce hormones, growth factors, including pleiotropic growth factors, cytokines, and chemokines, and to provide conditioned culture media for supporting the growth and expansion of other cell populations. In another aspect, the disclosure encompasses a lipo-derived lattice substantially devoid of cells, which includes extracellular matrix material form adipose tissue. The lattice can be used as a substrate to facilitate the growth and differentiation of cells, whether in vivo or in vitro, into anlagen or mature tissue or structures, as well as to provide an environment which maintains the viability of the cells.

The present disclosure thus provides methods and compositions for delivering incredibly large numbers of ASCs, precursors, or differentiated cells derived from adipose tissue for the procedures and treatments described herein. Additionally, for diseases that require cell infusions or administration, adipose tissue harvest is minimally invasive, yields many cells, and can be done repeatedly

The present disclosure encompasses the preparation and use of immortalized cell lines, including adipose tissue-derived cell lines capable of differentiating into at least one cell type. Various techniques for preparing immortalized cell lines are known to those of ordinary skill in the art.

Compositions comprising cells of the present disclosure can be employed in any suitable manner to facilitate the growth and differentiation of the desired tissue. For example, the composition can be constructed using three-dimensional or stereotactic modeling techniques. To direct the growth and differentiation of the desired structure, the composition can be cultured ex vivo in a bioreactor or incubator, as appropriate. In other examples, the structure is implanted within the host animal directly at the site in which it is desired to grow the tissue or structure. In still another example, the composition can be engrafted onto a host, where it will grow and mature until ready for use. Thereafter, the mature structure (or anlage) is excised from the host and implanted into the host, as appropriate.

One of ordinary skill in the art would appreciate that there are other carriers useful for delivering the cells of the present disclosure. Such carriers include calcium phosphate, hydroxyapatite, and synthetic or natural polymers such as collagen or collagen fragments in soluble or aggregated forms. In one example, such carriers serve to deliver the cells to a location or to several locations. In another example, the carriers and cells can be delivered either through systemic administration or by implantation. Implantation can be into one site or into several sites.

As indicated above, cells can be seeded onto and/or within the organic/inorganic composites of the present disclosure. Likewise, tissues such as cartilage can be associated with the composites prior to implantation within a patient. Examples of such cells include bone cells (such as osteoclasts, osteoblasts, and osteocytes), blood cells, epithelial cells, neural cells (e.g., neurons, astrocytes, and oligodendrocytes), and dental cells (odontoblasts and ameloblasts). Seeded cells can be autogenic, allogenic, or xenogeneic. Seeded cells can be encapsulated or non-encapsulated.

### Additional Uses

The pharmaceutical composition of the disclosure is useful as a cleanser. It is useful as a wound cleanser. It is also useful as a skin cleanser. In one aspect, the skin is injured or diseased. One of ordinary skill in the art will understand that the formulation used as a cleanser and its route and dosage of administration can be varied depending on the types of variables described herein.

### Kits

Also described are kits for improving vascular flow following thermal injury. The kit comprises an inhibitor of the decrease in vascular flow (or changes associated with a decrease in vascular flow) or a compound identified in the disclosure a standard, and an instructional material which describes administering the inhibitor or a composition comprising the inhibitor or compound to a cell or an animal. This should be construed to include other embodiments of kits that are known to those skilled in the art, such as a kit comprising a standard and a (preferably sterile) solvent suitable for dissolving or suspending the composition of the disclosure prior to administering the compound to a cell or an animal. Preferably, the animal is a mammal. More preferably, the mammal is a human.

As used herein, an "instructional material" includes a publication, a recording, a diagram, or any other medium of expression that can be used to communicate the usefulness of the compounds described herein in the kit for effecting alleviation of the various diseases or disorders recited herein. Optionally, or alternately, the instructional material may describe one or more methods of alleviating the diseases or disorders. The instructional material of the kit may, for example, be affixed to a container that contains a compound of the disclosure or be shipped together with a container that contains the compounds. Alternatively, the instructional material may be shipped separately from the container with the intention that the instructional material and the compound be used cooperatively by the recipient.

In accordance with the present disclosure, as described above or as discussed in the Examples below, there can be employed conventional chemical, cellular, histochemical, biochemical, molecular biology, microbiology, recombinant DNA, and clinical techniques which are known to those of skill in the art. Such techniques are explained fully in the literature. See for example, Sambrook et al., 1989 Molecular Cloning--a Laboratory Manual, Cold Spring Harbor Press; Glover, (1985) DNA Cloning: a Practical Approach; Gait, (1984) Oligonucleotide Synthesis; Harlow et al., 1988 Antibodies--a Laboratory Manual, Cold Spring Harbor Press; Roe et al., 1996 DNA Isolation and Sequencing: Essential Techniques, John Wiley; and Ausubel et al., 1995 Current Protocols in Molecular Biology, Greene Publishing.

### EXAMPLES

The invention is now described with reference to the following illustrative Examples.

A scald burn model of microvasculature in rat mesentery was developed. This tissue was chosen because of its ease of preparation and the ability to visually monitor dynamic microvascular changes, such as sludging and stasis, in real time under intravital microscopy. The exposed mesenteric microvessels were thermally injured before topical application of either Ringer's solution or Ringer's solution with 5% poloxamer-188.

### MATERIALS AND METHODS

### Rat Mesentery Model

All experiments and protocols were conducted in accordance with Animal Care and Use Committee guidelines. A rat mesenteric microvascular model, prepared similarly to previous studies, provided real-time observation of entire microvascular networks viewed en face.^{9,10} Blood flow within individual microvessels (arterioles, venules, and capillaries) was visualized clearly with magnification.

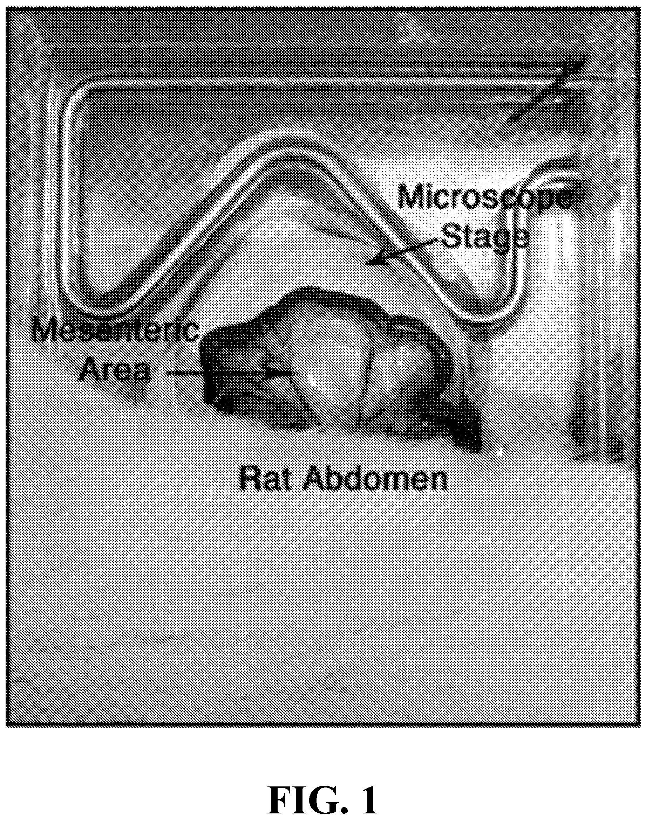

Male Sprague-Dawley rats (Harlan, Indianapolis, IN and Charles River, Boston, MA) weighing 450 g (±50 g) were anesthetized with an intramuscular injection of ketamine (80 mg/kg), xylazine (8 mg/kg), and atropine (0.08 mg/kg). The abdomen was shaved, sterilely prepped, and surgically incised. The animal remained warmed on a heating pad throughout the experiment. The ileum was gently removed from the abdomen and placed on a specifically designed plastic stage (Figure 1). Moistened gauze and polyethylene sheeting (Saran Premium Wrap, S.C. Johnson & Son, Inc, Racine, WI) protected the exposed mesentery during the experiment.

Ringer's solution was prepared according to the following formula: 137.9 mM NaCl, 4.7 mM KCl, 1.2 mM MgSO₄, and 1.9 mM CaCl₂. A continuous drip of either Ringer's solution (control) or 5% by weight poloxamer-188 in Ringer's solution (experiment) warmed to 37°C topically suffused the mesentery throughout the duration of the experiment.

### Intravital Microscopy

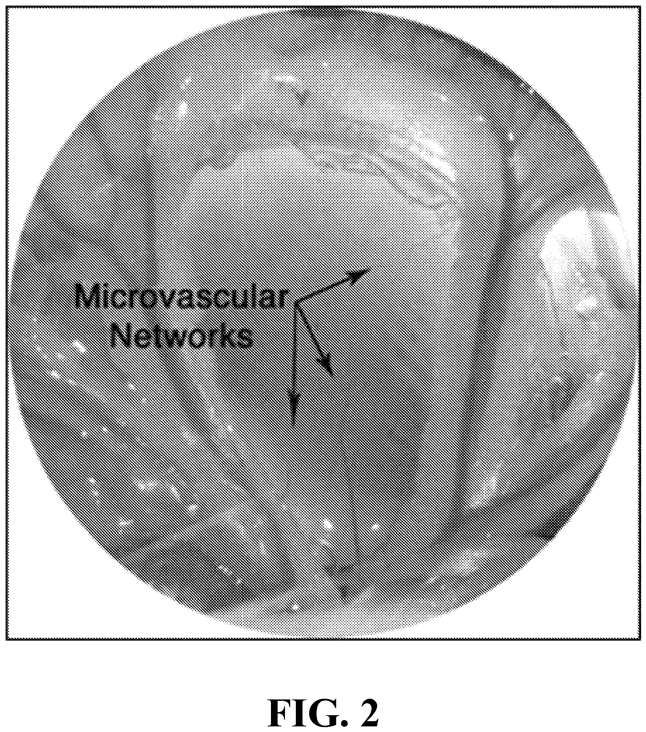

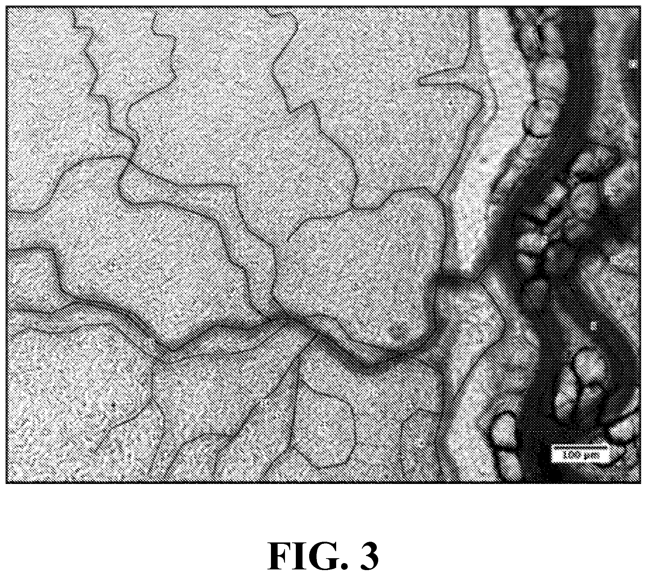

Areas of tissue containing microvascular networks were identified (Figure 2). Individual mesenteric windows (defined as an area of mesenteric microvascular network viewed by the microscope at one time) were examined using intravital microscopy under a 10x-objective in real time (0.3 DIC L/N1 WD 16.0 Nikon Eclipse 80i, Nikon, Tokyo, Japan). Total magnification, with the digital camera, was 280x. These locations were noted to allow return to the same area at a subsequent time (Figure 3). Six to nine windows per animal were identified and monitored at two time intervals: prethermal injury and 120 minutes postthermal injury.

### Thermal Injury

The mesentery was subjected to a controlled thermal burn injury by superfusing the tissue with 40 mL of Ringer's solution maintained at 55°C for 30 seconds. This protocol was modified from previously reported thermal injury models because of the increased thermal susceptibility of mesenteric tissue.^{2,11} Thermal injury initiated changes in the flow state of some of the microvessels, causing well-defined sludging and stasis.

### Image Capture and Analysis

During each monitored time interval, real-time video of the blood flow state within the microvasculature was captured using a digital camera (Olympus America, Center Valley, PA) mounted on the microscope. The video capture rate was 10 frames per second for 4 seconds per window. Transfer and storage of these video clips to a computer allowed postcollection analysis (Dell Optiplex GX 280 computer, Austin, TX; MicroFire software v1.0, Optronics, Goleta, CA).

Postcollection analysis included mapping each microvascular segment length and width (ImageJ software v1.36b, National Institutes of Health, Bethesda, MD) (Fig. 3). Microvascular width and blood flow direction allowed identification of individual microvessels as capillaries (5-10 µm) or venules (11-52 µm). Arterioles were excluded because they comprised less than 1% of the total microvasculature within the area framed by the windows.

A flow state (normal, sludging, or static) was assigned to each microvascular segment as viewed while replaying the video clips. Normal flow rates were assigned to vessels through which erythrocytes moved quickly and could not be identified as individual cells. Sludging occurred where erythrocytes bunched together, slowed to a rate allowing visualization of individual cells or intermittently stopped flowing. Static segments had no flowing erythrocytes within the microvascular lumen. The exact same microvessels within each window were assigned a flow state at 2 time intervals: before the thermal injury (prethermal injury) and then 120 minutes after the thermal injury (postthermal injury). The length of microvessels per tissue area (mm/mm2) was used as the standard metric. Microvessels contained within 24 windows obtained from 5 rats in the control group and 20 windows obtained from 3 rats in the poloxamer-188 treated group were analyzed.

### Statistical Analysts

All results are presented in the form of mean ± standard deviation. All comparisons were made using the statistical analysis tools proved by SigmaPlot 5.0 (Systat, Inc., Point Richmond, CA). Data were tested for normality and treatment group versus control comparisons were analyzed by the Student t test. Statistical significance was asserted by calculating an associated P value.

### RESULTS

Results were compiled for control and poloxamer-188-treated experiments. Capillaries and venules were compared separately between the two arms. Sludging and stasis were combined because they both represent abnormal flow states, which may contribute to ischemia or necrosis in injured tissue. Only microvessels noted to have normal flow before the thermal injury were included in the data analysis. A small number of microvessels (<1%) were noted to have abnormal flow before thermal injury and were excluded from the analysis.
The total length of normally flowing capillaries was compared with that of capillaries containing sludged or static flow for both the control and poloxamer-188-treated groups. The flow states were compared at 120 minutes after thermal injury (Fig. 4). After control treatment, there was no significant difference between the total length of normally flowing and abnormally flowing (i.e., sludged or static) capillary microvessels. This means that about one half of the vessels converted from a normal flow state (before thermal injury) to a sludged or static state 120 minutes after thermal injury. However, after poloxamer-188 treatment, the total length of normally flowing vessels was greater than the total length of abnormally flowing vessels. That is, fewer vessels converted to abnormal flow states after thermal injury.

A comparison of the total length of venules that contained normal blood flow versus abnormal flow revealed a similar effect with poloxamer-188 treatment (Fig. 5). The control treatment demonstrated a statistically similar number of venules with normal and abnormal flows after 120 minutes. After poloxamer-188 treatment, the resulting total length of normal flow vessels was greater than venules with sludging or stasis 120 minutes after thermal injury. This result suggests that poloxamer-188 treatment demonstrated a statistically significant reduction in the amount of blood sludging and stasis.

The total length of sludged or static microvessels 120 minutes after thermal injury also is reported as a percentage of total observed vessels (Fig. 6). The percentage of abnormally flowing capillaries in the control group was 62% versus only 23% in the poloxamer-188-treated group, a statistically significant difference. Venules demonstrated a similar result. Abnormal flow was noted in 54% of observed venules in the control-treated group. However, only 32% of the poloxamer-188-treated venules demonstrated abnormal flow 120 minutes after thermal injury.

### DISCUSSION

Thermal injuries most commonly occur at dermal levels. Topical treatment of these injuries is a basic tenet of burn care. A topical burn treatment, which has the potential to decrease the total area of tissue loss could improve the care of these injuries.

The microvascular changes in the zone of stasis in a burn have been a topic of intense research activity. Abnormal microvascular blood flow can contribute to ischemia of thermally injured tissue. Flow changes of sludging or stasis within microvessels are early signs of injury. Therefore, decreasing the amount of microvascular sludging or stasis may decrease the area of tissue loss after thermal injury.

This study was designed to evaluate the effect of a topical agent on microvascular flow of thermally injured tissue. Poloxamer-188 is a nonionic, amphiphilic, water-soluble triblock copolymer (polyoxyethylene-polyoxypropylene) structure. Properties of this copolymer allow it to act as a surfactant in blood, increasing whole blood clot permeability and fibrinolysis.⁵⁻⁷

The model for this study was developed to allow direct, real-time visualization of the changes in the microvasculature within the thermal injury field. The rat mesentery provides ready access to large networks of microvessels. Blood flow through these can be monitored directly over a period of time using intravital microscopy. One limitation of the model is the amount of observation time the model permits. Two hours is sufficient to appreciate changes in microvascular blood flow after thermal injury. However, these changes may continue for a much longer time period than can be assessed with the current model.

All microvessels included in the analysis were noted to have normal flow before the thermal injury. Sludging and stasis within the microvessels was considered abnormal blood flow at the endpoint of 120 minutes after thermal injury. This type of flow was compared with normal flow to assess changes over time that thermal injury can cause. Video recordings of the microvascular flow allowed careful comparison of flow states of the same microvascular segments after 120 minutes.

Overall, a similar number of windows (and a similar total length per unit area of capillaries and venules) were compared between the control group and the poloxamer-188 group. For both capillaries and venules, the control group demonstrated similar lengths per unit area of normally and abnormally flowing vessels at the end point. However, there was a difference between the total length per area of normally flowing vessels and those that demonstrated sludging or stasis for poloxamer-188-treated capillaries and venules (Figs. 4 and 5). The total length per unit area of microvessels was less in the abnormally flowing (sludged or static) groups, reaching statistical significance in the venule comparison. This is an indication that the poloxamer-188 may have exerted a physiological effect on the microvessels that kept fewer from converting to sludging or static flow states during the time period studied.

In addition, there was a noted decrease in the percentage of sludged or static vessels (both capillaries and venules) when treated with poloxamer-188. The decrease from 62 to 23% for capillaries is statistically significant (Fig. 6A). Again, poloxamer-188 appears to have an effect, which reduces or prevents the microvascular changes caused by thermal injury. A similar decrease in the percentage of venular sludging or stasis is noted with the application of poloxamer-188, though significance was not quite reached (Fig. 6B).

Although, this study demonstrates differences in flow within mesenteric microvessels, conversion to a dermal model study is required to confirm the benefits of poloxamer-188 as a topical treatment. Also, the correlation between improving flow in the zone of stasis and eventual tissue preservation needs to be more fully understood.

Despite decades of research investigating poloxamer-188, there is more to be known about its mechanism of action. This includes whether it is absorbed into the blood when it is applied topically, whether it impacts microvascular permeability or vascular tone, or whether it affects the osmotic environment of the interstitial tissue.

Overall, the results of this study show, for the first time, that topical delivery of poloxamer-188 reduces microvascular stasis and sludging after thermal injury.

This may eventually lead to a proven benefit to thermally injured tissue by the topical treatment with poloxamer-188.

### BIBLIOGRAPHY

1. Jackson DM. The diagnosis of the depth of burning. Br J Surg. 1953;40:588-596.
2. Boykin JV, Eriksson E, Pittman RN. In vivo microcirculation of a scald burn and the progression of postburn dermal ischemia. Plast Reconstr Surg. 1980;66:191-198.
3. Zawacki BE. Reversal of capillary stasis and prevention of necrosis in bums. Ann Surg. 1975;180:98-102.
4. Isik S, Unal S, Sefettin I, et al. Saving the zone of stasis in bums with recombinant tissue-type plasminogen activator (r-tPA): an experimental study in rats. Bums. 1998;24:217-223.
5. Grove FL, Kahn RS, Heron MW, et al. A nonionic surfactant and blood viscosity: experimental observations. Arch Surg. 1973;3:307-310.
6. Hunter RL, Bennett B, Check IJ. The effect of poloxamer 188 on the rate of in vitro thrombolysis mediated by t-PA and streptokinase. Fibrinolysis. 1990;4:117-123.
7. Carr ME Jr, Powers PL, Jones MR. Effects of poloxamer 188 on the assembly, structure, and dissolution of fibrin clots. Thromb Haemost. 1991;66:565-568.
8. Baskaran H, Toner M, Yarmush ML, et al. Poloxamer-188 improves capillary blood flow and tissue viability in a cutaneous burn wound. J Surg Res. 2001;101:56-61.
9. Murfee WL, Rehorn MR, Peirce SM, et al. Perivascular cells along venules upregulate NG2 expression during microvascular remodeling. Microcirculation. 2006;13:261-273.
10. Sato M, Ohshima N. Effect of wall shear rate on thrombogenesis in microvessels of rat mesentery. Circ Res. 1990;66:941-949.
11. Aggarwal SJ, Da Costa R, Diller KR, et al. The effects of burn injury on vasoactivity in hamster peripheral microcirculation. Microvasc Res. 1990;40:73-87.

Headings are included herein for reference and to aid in locating certain sections. These headings are not intended to be limiting.

## Claims

1. A topical pharmaceutical composition comprising an effective amount of at least one surface active copolymer and optionally at least one additional therapeutic agent, wherein said at least one surface active copolymer is poloxamer 188 at 5% concentration, for use in the treatment of an injury, disease, or disorder **characterised by** decreased blood flow;
wherein the said injury, disease, or disorder is selected from the group consisting of thermal injury, skin injury, soft tissue injury, non-healing skin wound, burns, acute wound, chronic wound, scrape, cut, incision, laceration, decubitis, pressure ulcer, chronic venous ulcer, venous stasis ulcer, diabetic ulcer, arterial ulcer, radiation ulcer, traumatic wound, open complicated non-healing wound, body piercing, bite wound, insect bite, insect sting, stab wound, gunshot wound, stretch injury, crush wound, compression wound, fracture, sprain, strain, stroke, infarction, aneurism, herniation, ischemia, fistula, dislocation, radiation, surgery, cell, tissue or organ grafting, and cancer.

2. The topical composition for use according to claim 1, wherein said injury is a thermal injury, preferably wherein said thermal injury is a cutaneous injury or an injury of the mesentery of the intestine.

3. The topical composition for use according to claim 1, wherein said burn is selected from the group of burns consisting of thermal, radiation, chemical, electrical, steam, and sunburn.

4. The topical composition for use according to claim 1, wherein said pharmaceutical composition comprises at least two surface active copolymers.

5. The topical composition for use according to claim 1, wherein said at least one additional therapeutic agent is selected from the group consisting of aspirin, pentoxifylline, and clopidogrel bisulfate, anesthetic, analgesic, antimicrobial, steroid, growth factor, cytokine, and anti-inflammatory agents.

6. The topical composition for use according to claim 5, wherein said at least one additional therapeutic agent is an anesthetic selected from the group consisting of benzocaine, lidocaine, bupivocaine, dibucaine, mepivocaine, etidocaine, tetracaine, butanilicaine, and trimecaine.

7. The topical composition for use according to claim 5, wherein at least one of said additional therapeutic agents is an antimicrobial agent, preferably wherein said antimicrobial agent is selected from the group consisting of antibacterial, antifungal, and antiviral agents, or wherein the antimicrobial agent is selected from the group consisting of silver sulfadiazine, nystatin, nystatin/triamcinolone, bacitracin, nitrofurazone, nitrofurantoin, a polymyxin, doxycycline, antimicrobial peptides, beosporin, polysporin, silver salts, iodine, benzalkonium chloride, alcohol, hydrogen peroxide, and chlorhexidine.

8. The topical composition for use according to claim 1, wherein the pharmaceutical composition is formulated as a gel, preferably wherein said gel is a stable gel.

9. The topical composition for use according to claim 1, wherein said decreased blood flow is in blood vessels having a diameter from 5 µm to 100 µm, preferably wherein said blood vessels have a diameter from 10 µm to 50 µm.

10. The topical composition for use according to claim 1, wherein the size of said at least one surface active copolymer ranges from an Mn of 600 to 20,000, preferably wherein the size of said at least one surface active copolymer ranges from an Mn of 1,000 to 10,000.

## Patentansprüche

1. Eine topische pharmazeutische Zusammensetzung, beinhaltend eine wirksame Menge mindestens eines oberflächenaktiven Copolymers und optional mindestens eines zusätzlichen Therapeutikums, wobei das mindestens eine oberflächenaktive Copolymer Poloxamer 188 in einer Konzentration von 5 % ist, zur Verwendung bei der Behandlung einer Verletzung, Erkrankung oder Störung, **gekennzeichnet durch** verringerten Blutfluss;
wobei die Verletzung, Erkrankung oder Störung aus der Gruppe ausgewählt ist, die aus Folgendem besteht: thermischer Verletzung, Hautverletzung, Weichgewebeverletzung, nicht heilender Hautwunde, Verbrennungen, akuter Wunde, chronischer Wunde, Schürfwunde, Schnittwunde, Inzision, Platzwunde, Decubitis, Druckgeschwür, chronischem venösem Geschwür, venösem Stauungsgeschwür, Diabetesgeschwür, arteriellem Geschwür, Strahlungsgeschwür, traumatischer Wunde, offener komplizierter nicht heilender Wunde, Piercing, Bisswunde, Insektenbiss, Insektenstich, Stichwunde, Schusswunde, Dehnungsverletzung, Quetschwunde, Stauchwunde, Bruch, Verstauchung, Muskelzerrung, Schlaganfall, Infarkt, Aneurysma, Herniation, Ischämie, Fistel, Dislozierung, Strahlung, Operation, Zell-, Gewebe- oder Organtransplantation und Krebs.

2. Topische Zusammensetzung zur Verwendung gemäß Anspruch 1, wobei die Verletzung eine thermische Verletzung ist, wobei die thermische Verletzung vorzugsweise eine kutane Verletzung oder eine Verletzung des Gekröses des Darms ist.

3. Topische Zusammensetzung zur Verwendung gemäß Anspruch 1, wobei die Verbrennung aus der Gruppe von Verbrennungen ausgewählt ist, die aus thermischer Verbrennung, Strahlungsverbrennung, chemischer Verbrennung, elektrischer Verbrennung, Dampfverbrennung und Sonnenbrand besteht.

4. Topische Zusammensetzung zur Verwendung gemäß Anspruch 1, wobei die pharmazeutische Zusammensetzung mindestens zwei oberflächenaktive Copolymere beinhaltet.

5. Topische Zusammensetzung zur Verwendung gemäß Anspruch 1, wobei das mindestens eine zusätzliche Therapeutikum aus der Gruppe ausgewählt ist, die aus Folgendem besteht: Aspirin, Pentoxifyllin und Clopidogrelbisulfat, Anästhetikum, Analgetikum, antimikrobiellem Wirkstoff, Steroid, Wachstumsfaktor, Cytokin und entzündungshemmenden Mitteln.

6. Topische Zusammensetzung zur Verwendung gemäß Anspruch 5, wobei das mindestens eine zusätzliche Therapeutikum ein Anästhetikum ist, ausgewählt aus der Gruppe, bestehend aus Benzocain, Lidocain, Bupivocain, Dibucain, Mepivocain, Etidocain, Tetracain, Butanilicain und Trimecain.

7. Topische Zusammensetzung zur Verwendung gemäß Anspruch 5, wobei mindestens eines der zusätzlichen Therapeutika ein antimikrobielles Mittel ist, wobei das antimikrobielle Mittel vorzugsweise aus der Gruppe ausgewählt ist, die aus antibakteriellen, antimykotischen und antiviralen Mitteln besteht, oder wobei das antimikrobielle Mittel aus der Gruppe ausgewählt ist, die aus Folgendem besteht:
Silbersulfadiazin, Nystatin, Nystatin/Triamcinolon, Bacitracin, Nitrofurazon, Nitrofurantoin, einem Polymyxin, Doxycyclin, antimikrobiellen Peptiden, Beosporin, Polysporin, Silbersalzen, Iod, Benzalkoniumchlorid, Alkohol, Wasserstoffperoxid und Chlorhexidin.

8. Topische Zusammensetzung zur Verwendung gemäß Anspruch 1, wobei die pharmazeutische Zusammensetzung als ein Gel formuliert ist, wobei das Gel vorzugsweise ein stabiles Gel ist.

9. Topische Zusammensetzung zur Verwendung gemäß Anspruch 1, wobei der verringerte Blutfluss in Blutgefäßen stattfindet, die einen Durchmesser von 5 µm bis 100 µm aufweisen, wobei die Blutgefäße vorzugsweise einen Durchmesser von 10 µm bis 50 µm aufweisen.

10. Topische Zusammensetzung zur Verwendung gemäß Anspruch 1, wobei die Größe des mindestens einen oberflächenaktiven Copolymers von einem Mn von 600 bis 20 000 reicht, wobei die Größe des mindestens einen oberflächenaktiven Copolymers vorzugsweise von einem Mn von 1 000 bis 10 000 reicht.

## Revendications

1. Une composition pharmaceutique topique comprenant une quantité efficace d'au moins un copolymère tensioactif et facultativement au moins un agent thérapeutique supplémentaire, dans laquelle ledit au moins un copolymère tensioactif est du poloxamère 188 à une concentration de 5 %, pour son utilisation dans le traitement d'une lésion, d'une maladie, ou d'un trouble caractérisé(e) par une diminution du flux sanguin ;
dans laquelle ladite lésion, ladite maladie, ou ledit trouble est sélectionné(e) dans le groupe constitué d'une lésion thermique, une lésion de la peau, une lésion des tissus mous, une plaie de la peau non cicatrisante, des brûlures, une plaie aiguë, une plaie chronique, une égratignure, une coupure, une incision, une lacération, une escarre de décubitus, de pression, un ulcère veineux chronique, un ulcère de stase veineux, un ulcère diabétique, un ulcère artériel, un ulcère roentgenien, une plaie traumatique, une plaie non cicatrisante compliquée ouverte, un piercing corporel, une plaie par morsure, une morsure d'insecte, une piqûre d'insecte, une plaie par arme blanche, une plaie par arme à feu, une lésion par étirement, une plaie d'écrasement, une plaie de compression, une fracture, une entorse, une foulure, un accident vasculaire cérébral, un infarctus, un anévrisme, une herniation, une ischémie, une fistule, une dislocation, une radiation, une intervention chirurgicale, une greffe de cellule, de tissu ou d'organe, et le cancer.

2. La composition topique pour son utilisation selon la revendication 1, dans laquelle ladite lésion est une lésion thermique, préférablement dans laquelle ladite lésion thermique est une lésion cutanée ou une lésion du mésentère de l'intestin.

3. La composition topique pour son utilisation selon la revendication 1, dans laquelle ladite brûlure est sélectionnée dans le groupe de brûlures constitué de brûlures thermique, par radiation, chimique, électrique, par la vapeur, et par le soleil.

4. La composition topique pour son utilisation selon la revendication 1, ladite composition pharmaceutique comprenant au moins deux copolymères tensioactifs.

5. La composition topique pour son utilisation selon la revendication 1, dans laquelle ledit au moins un agent thérapeutique supplémentaire est sélectionné dans le groupe constitué de l'aspirine, de la pentoxifylline, et du bisulfate de clopidogrel, d'anesthésique, d'analgésique, d'antimicrobien, de stéroïde, de facteur de croissance, de cytokine, et d'agents anti-inflammatoires.

6. La composition topique pour son utilisation selon la revendication 5, dans laquelle ledit au moins un agent thérapeutique supplémentaire est un anesthésique sélectionné dans le groupe constitué de la benzocaïne, de la lidocaïne, de la bupivocaïne, de la dibucaïne, de la mépivocaïne, de l'étidocaïne, de la tétracaïne, de la butanilicaïne, et de la trimécaïne.

7. La composition topique pour son utilisation selon la revendication 5, dans laquelle au moins l'un desdits agents thérapeutiques supplémentaires est un agent antimicrobien, préférablement dans laquelle ledit agent antimicrobien est sélectionné dans le groupe constitué d'agents antibactériens, antifongiques, et antiviraux, ou dans laquelle l'agent antimicrobien est sélectionné dans le groupe constitué de la sulfadiazine d'argent, de la nystatine, de la nystatine/triamcinolone, de la bacitracine, de la nitrofurazone, de la nitrofurantoïne, d'une polymyxine, de la doxycycline, de peptides antimicrobiens, de la béosporine, de la polysporine, de sels d'argent, de l'iode, du chlorure de benzalkonium, de l'alcool, du peroxyde d'hydrogène, et de la chlorhexidine.

8. La composition topique pour son utilisation selon la revendication 1, la composition pharmaceutique étant formulée sous la forme d'un gel, ledit gel étant préférablement un gel stable.

9. La composition topique pour son utilisation selon la revendication 1, dans laquelle ladite diminution de flux sanguin est dans des vaisseaux sanguins présentant un diamètre allant de 5 µm à 100 µm, lesdits vaisseaux sanguins présentant préférablement un diamètre allant de 10 µm à 50 µm.

10. La composition topique pour son utilisation selon la revendication 1, dans laquelle la taille dudit au moins un copolymère tensioactif est comprise dans la gamme d'une Mn allant de 600 à 20 000, préférablement dans laquelle la taille dudit au moins un copolymère tensioactif est comprise dans la gamme d'une Mn allant de 1 000 à 10 000.
